# EUROPEAN PATENT SPECIFICATION

(11) **EP 3 672 605 B1**
(45) Date of publication and mention of the grant of the patent: **23.10.2024**
(21) Application number: 18773306.8
(22) Date of filing: 23.08.2018
(51) Int. Cl.: A61K 35/28, A61K 48/00

(54) **IN UTERO TRANSPLANTATION OF FACTOR VIII-EXPRESSING CELLS FOR TREATMENT OF HEMOPHILIA**
TRANSPLANTATION VON FAKTOR VIII-EXPRIMIERENDEN ZELLEN IN DER GEBÄRMUTTER ZUR BEHANDLUNG VON HÄMOPHILIE
TRANSPLANTATION IN UTERO DE CELLULES EXPRIMANT LE FACTEUR VIII POUR LE TRAITEMENT DE L'HÉMOPHILIE

(30) Priority: 23.08.2017 US 201762549298 P
(43) Date of publication of application: 01.07.2020
(73) Proprietor: Wake Forest University Health Sciences, Winston-Salem, North Carolina 27157 (US); Emory University, Atlanta, Georgia 30322 (US); Children's Healthcare Of Atlanta, Inc., Atlanta, GA 30329 (US)
(72) Inventor: ALMEIDA-PORADA, Maria, Graca, N., D., Winston-Salem NC 27106 (US); PORADA, Christopher, D., Winston-Salem NC 27106 (US); ATALA, Anthony, Winston-Salem NC 27104 (US); DOERING, Christopher, B., Atlanta GA 30345 (US); SPENCER, H., Trent, Marietta GA 30062 (US)
(74) Representative: Forresters IP LLP
(86) International application number: PCT/US2018/047751
(87) International publication number: WO 2019/040747

(56) References cited:
- WO-A2-00/29002
- WO-A2-2016/183593
- XIAN-YANG ZHANG ET AL: "Lentiviral Vectors for Sustained Transgene Expression in Human Bone Marrow?Derived Stromal Cells", MOLECULAR THERAPY, vol. 5, no. 5, 1 May 2002 (2002-05-01), US, pages 555 - 565, XP055519976, ISSN: 1525-0016, DOI: 10.1006/mthe.2002.0585
- KUMAR PRIYADARSINI ET AL: "In Utero Transplantation of Placenta-Derived Mesenchymal Stromal Cells for Potential Fetal Treatment of Hemophilia A", CELL TRANSPLANTATION, vol. 27, no. 1, Sp. Iss. SI, January 2018 (2018-01-01), pages 130 - 139, XP002786326
- CHRISTOPHER D PORADA ET AL: "Phenotypic correction of hemophilia A in sheep by postnatal intraperitoneal transplantation of FVIII-expressing MSC", EXPERIMENTAL HEMATOLOGY, vol. 39, no. 12, 7 September 2011 (2011-09-07), pages 1124 - 1135.e4, XP028112104, ISSN: 0301-472X, [retrieved on 20110908], DOI: 10.1016/J.EXPHEM.2011.09.001
- CHRISTOPHER D. PORADA ET AL: "Treatment of Hemophilia A in Utero and Postnatally using Sheep as a Model for Cell and Gene Delivery", JOURNAL OF GENETIC SYNDROMES & GENE THERAPY, vol. S1, no. 02, 1 January 2013 (2013-01-01), XP055518977, DOI: 10.4172/2157-7412.S1-011
- I. NAZAROV ET AL: "Multipotent Stromal Stem Cells from Human Placenta Demonstrate High Therapeutic Potential", STEM CELLS TRANSLATIONAL MEDICINE, vol. 1, no. 5, 1 May 2012 (2012-05-01), pages 359 - 372, XP055283323, ISSN: 2157-6564, DOI: 10.5966/sctm.2011-0021
- CHRISTOPHER D. PORADA ET AL: "Hemophilia A: an ideal disease to correct in utero", FRONTIERS IN PHARMACOLOGY, vol. 5, 11 December 2014 (2014-12-11), XP055521842, DOI: 10.3389/fphar.2014.00276
- WANG Q ET AL: "The Mesenchymal Stem Cells Derived from Transgenic Mice Carrying Human Coagulation Factor VIII Can Correct Phenotype in Hemophilia A Mice", JOURNAL OF GENETICS AND GENOMICS, ELSEVIER LTD, AMSTERDAM, NL, vol. 40, no. 12, 20 December 2013 (2013-12-20), pages 617 - 628, XP002785325, ISSN: 1673-8527, [retrieved on 20131126], DOI: 10.1016/J.JGG.2013.11.002
- N. J. WARD ET AL: "Codon optimization of human factor VIII cDNAs leads to high-level expression", BLOOD, vol. 117, no. 3, 20 January 2011 (2011-01-20), pages 798 - 807, XP055052195, ISSN: 0006-4971, DOI: 10.1182/blood-2010-05-282707
- KERRY L. DOORISS ET AL: "Comparison of Factor VIII Transgenes Bioengineered for Improved Expression in Gene Therapy of Hemophilia A", HUMAN GENE THERAPY, vol. 20, no. 5, 1 May 2009 (2009-05-01), pages 465 - 478, XP055136766, ISSN: 1043-0342, DOI: 10.1089/hum.2008.150
- SOKAL EM ET AL: "Mesenchymal stem cell treatment for hemophilia: a review of current knowledge", JOURNAL OF THROMBOSIS AND HAEMOSTASIS, JOHN WILEY & SONS, vol. 13, no. Suppl. 1, 1 June 2015 (2015-06-01), pages S161 - S166, XP002785324, ISSN: 1538-7836, [retrieved on 20150619], DOI: 10.1111/JTH.12933

## Description

### STATEMENT AS TO RIGHTS TO INVENTIONS MADE UNDER FEDERALLY SPONSORED RESEARCH AND DEVELOPMENT

This invention was made with government support under grant number 1R01HL135853-01A1 awarded by the U.S. National Institutes of Health (NIH). The government has certain rights in the invention.

### CROSS REFERENCE TO RELATED APPLICATION

This application claims the benefit of U.S. Provisional Application Serial No. 62/549,298, filed August 23, 2017.

### REFERENCE TO A SEQUENCE LISTING SUBMITTED AS A TEXT FILE VIA EFS-WEB

The official copy of the sequence listing is submitted electronically via EFS-Web as an ASCII formatted sequence listing with a file named SEQ_WFIRM17-915.txt, created on August 23, 2018, and having a size of 176 KB and is filed concurrently with the specification. The sequence listing contained in this ASCII formatted document is part of the specification.

### BACKGROUND

Factor VIII is an essential blood clotting factor. The protein circulates in the bloodstream in an inactive form, bound to another molecule called von Willebrand factor, until an injury that damages blood vessels occurs. In response to injury, coagulation factor VIII is activated and separates from von Willebrand factor. The active protein interacts with another coagulation factor called Factor IX. This interaction sets off a chain of additional chemical reactions that form a blood clot.

Hemophilia A (HA) is the most common inheritable coagulation deficiency, affecting 1 in 5000 boys, approximately 60% of whom present with the severe form of the disease. Mutations in the Factor VIII gene that result in decreased or defective Factor VIII protein give rise to HA, a recessive X-linked disorder. Individuals with severe HA experience recurrent hematomas of subcutaneous connective tissue/muscle, internal bleeding, and frequent hemarthrosis, leading to chronic debilitating arthropathies. Current treatment is frequent infusions of Factor VIII (plasma-derived or recombinant) to maintain hemostasis, which greatly improves quality of life for many HA patients. While current therapeutic products for HA offer reliable prophylactic and therapeutic efficacy, they are very expensive and do not cure the underlying disease, thus requiring administration for the entire life of the patient. In addition, more than 30% of patients with severe HA develop inhibitory antibodies to the infused Factor VIII therapeutic, placing them in danger of treatment failure. This is a significant and serious complication/challenge in the clinical management/treatment of HA. While protein-based immune tolerance induction (ITI) therapy has been used with some success in this patient group, its cost extends into the millions of dollars per patient, it is only effective in about 60% of patients, and its mechanism of action is largely unknown. These shortcomings with existing therapy for patients who develop inhibitors highlight the need for innovative approaches to surmount this immunological hurdle.

### BRIEF SUMMARY

The invention is defined by the claims. In one aspect, provided modified mensenchymal stem/stromal cells (MSC) for use in methods of treating a subject prenatally diagnosed as having hemophilia A, the method involving injecting modified MSC into the subject *in utero,* wherein the modified MSC are modified, via introduction of a transgene or genome editing, to express high levels of Factor VIII protein or high levels of both Factor VIII protein and von Willebrand factor (vWF) protein, wherein the Factor VIII gene sequence comprises one or more genetic modifications that increase protein expression, protein stability, or both, and wherein the modified MSC are c-kit expressing modified placental tissue MSC.

The above described and many other features and attendant advantages of embodiments of the present disclosure will become apparent and further understood by reference to the following detailed description when considered in conjunction with the accompanying drawings.

### BRIEF DESCRIPTION OF THE DRAWINGS

**FIG. 1** shows the complete absence of any FVIII antigen/cross-reactive material (CRM) in the hemophilia A sheep model according to some aspects of the disclosure.
**FIG. 2** shows a schematic diagram of an ovine Factor VIII transgene expression vector according to some aspects of the disclosure.
**FIG. 3** shows a schematic diagram of an ET3 Factor VIII transgene expression vector according to some aspects of the disclosure.
**FIG. 4** shows a schematic diagram of a human Factor VIII transgene expression vector according to some aspects of the disclosure.
**FIG. 5** shows a schematic flow diagram for producing modified MSC in which the MSC are isolated from placental tissue according to some aspects of the disclosure.
**FIG. 6A** shows assessment of phenotypic markers in PLCs according to some aspects of the disclosure.
**FIG. 6B** shows flow cytometric analysis of PLC constitutively expressed levels of FVIII protein according to some aspects of the disclosure.
**FIG. 6C** shows assessment of normalized levels of PLC constitutively expressed levels of FVIII protein according to some aspects of the disclosure.
**FIG. 7A** shows assessment of normalized levels of secretion of FVIII protein by PLC engineered/transduced to express high levels of FVIII according to some aspects of the disclosure.
**FIG. 7B** shows assessment of secretion of FVIII protein by transduced as compared to non-transduced PLCs according to some aspects of the disclosure.
**FIGS. 8A-8C** show assessment of phenotypic markers in transduced as compared to non-transduced PLCs according to some aspects of the disclosure.
**FIG. 9A** shows assessment of expression of Toll-like receptors (TLRs) in mcoET3-transduced PLCs as compared to non-transduced PLCs according to some aspects of the disclosure.
**FIG. 9B** shows assessment of expression of stress molecules in mcoET3-transduced PLCs as compared to non-transduced PLCs according to some aspects of the disclosure.
**FIG. 10** shows assessment of normalized levels of PLC secretion of FVIII protein by non-transduced-PLC, IcoHSQ-PLC, lcoET3-PLC, ET3-PLC, and mcoET3-PLC according to some aspects of the disclosure.
**FIG. 11A** shows assessment of plasma FVIII activity levels measured by aPTT in sheep at intervals from 1-14 months post-prenatal transplantation with mcoET3-PLC according to some aspects of the disclosure.
**FIG. 11B** shows the growth curve by weight (kg) of lambs following prenatal transplantation of mcoET3-PLC according to some aspects of this disclosure.
**FIG. 12** shows ET3 and human FVIII expression in tissues of lambs following prenatal transplantation of mcoET3-PLC according to some aspects of this disclosure.
**FIG. 13** shows the relative percentage of cells expressing ET3 in tissues of lambs following prenatal transplantation of mcoET3-PLC according to some aspects of this disclosure.

### DETAILED DESCRIPTION

Provided in this disclosure are modified mensenchymal stem/stromal cells (MSC) for use in methods of treatment for subjects having hemophilia A. The methods are prenatal therapies comprising administering to a subject with hemophilia A MSC that have been modified, via introduction of a transgene or genome editing, to express high levels of Factor VIII protein or high levels of both Factor VIII protein and von Willebrand factor (vWF) protein, wherein the Factor VIII gene sequence comprises one or more genetic modifications that increase protein expression, protein stability, or both, and wherein the modified MSC are c-kit expressing modified placental tissue MSC. Provided methods are effective as first-line therapies for subjects that have been diagnosed prenatally. The MSC are modified to express high levels of Factor VIII protein. In some instances, the MSC are modified to express high levels of Factor VIII and high levels of another protein, such as von Willebrand factor. The MSC may be modified by the introduction of a transgene (for example, using a viral vector) or via genome-editing (for example, using the CRISPR/Cas9 system). Administering the modified MSC to the subject results in engraftment of the modified cells. The engrafted cells produce Factor VIII on a continuing basis in the subject and provide long-lasting (ideally lifelong) therapeutic benefit to the subject by promoting blood coagulation. The MSC used in the methods are isolated from biological samples obtained prenatally or from unrelated individuals. Because the fetal immune system is not developed, a fetus is tolerogenic, and the cells used in the method do not have to be from the subject (fetus) being treated (the cells can be allogeneic).

In one aspect, provided is a mesenchymal stem/stromal cells (MSC) for use in a method of treating a subject prenatally diagnosed as having hemophilia A comprising injecting modified MSC into the subject *in utero,* wherein the modified MSC are modified via introduction of a transgene or genome, to express high levels of Factor VIII protein or high levels of both Factor VIII protein and von Willebrand factor (vWF) protein, wherein the Factor VIII gene sequence comprises one or more genetic modifications that increase protein expression, protein stability, or both, and wherein the modified MSC are c-kit expressing modified placental tissue MSC. Intrauterine transplantation (IUTx) is a clinically viable procedure, which has safely been performed for decades in humans. It is one of the few therapies that can promise the birth of a healthy infant. To-date, IUTx has been performed in close to 50 patients for 14 different genetic disorders. The provided methods are an IUTx-based strategy to treat HA and induce immune tolerance to Factor VIII. The methods lead to sustained levels of FVIII that are curative or at least sufficient to convert severe HA to a mild phenotype.

As used herein the terms treatment, treat, or treating refer to a method of reducing one or more symptoms of a disease or condition. In some instances, treatment results in a 5%, 10%, 20%, 30%, 40%, 50%, 60%, 70%, 80%, 90%, or 100% reduction in the severity of one or more symptoms of the disease or condition. In some instances, treatment results in at least a 60%, 65%, 70%, 75%, 80%, 85%, 90%, or 95% reduction in the severity of one or more symptoms of the disease or condition. In some instances, treatment results in a 100% reduction in the severity of one or more symptoms of the disease or condition. For example, a method for treating a disease is considered to be a treatment if there is a 5% reduction in one or more symptoms or signs of the disease in a subject as compared to a control. As used herein, control refers to the untreated condition. In some instances, the reduction can be a 5%, 10%, 20%, 30%, 40%, 50%, 60%, 70%, 80%, 90%, 100%, or any percent reduction in between 10% and 100% as compared to native or control levels. In some instances, the reduction can be at least a 65%, 70%, 75%, 80%, 85%, 90%, or 95% reduction as compared to native or control levels. In some instances, the reduction can be a 100% reduction. It is understood that treatment does not necessarily refer to a cure or complete ablation of the disease, condition, or symptoms of the disease or condition. As used herein, references to decreasing, reducing, or inhibiting include a change of 5%, 10%, 20%, 30%, 40%, 50%, 60%, 70%, 80%, 90% or greater as compared to a control level. Such terms can include, but do not necessarily include, complete elimination.

The subject on which the method is performed has been diagnosed clinically or genetically with hemophilia A. The subject is mammalian, including humans; non-human primates, such as apes and monkeys; cattle; horses; sheep; rats; dogs; cats; mice; pigs; and goats. In some embodiments, the subject is a human, a dog, a horse, a sheep, a cow, or a cat. The subject may be male or female. The subject is a subject in utero; that is, a subject that has not yet been born. Where the subject is human, the subject is a human fetus between 12 and 22 weeks of gestation. For example, the fetus may be at 12-16 weeks, 16-18 weeks, 18-20 weeks, 20-22 weeks, 12-18 weeks, 12-20 weeks, 16-20 weeks, 16-22 weeks, 12-15 weeks, 13-17 weeks, or 17-21 weeks gestation. As a recessive X-linked disorder, a male subject will carry an X chromosome that has a mutation in the Factor VIII gene. A female subject that has hemophilia A will either have a mutated Factor VIII allele on both X chromosomes or will have a mutant Factor VIII allele on one X chromosome and have an inactive normal Factor VIII allele on the other X chromosome. In some instances, the subject may be a female carrier of hemophilia A that has a mutant Factor VIII allele on one X chromosome and a normal Factor VIII gene on the other X chromosome. Such carrier females may be symptomatic carriers that exhibit bleeding disorder symptoms. Subjects may be diagnosed via prenatal genetic testing, particularly in instances where there is a family history of hemophilia. The DNA from biological samples obtained from amniocentesis, chorionic villi sampling, or cell-free fetal DNA present in maternal peripheral blood may be analyzed for mutations in the Factor VIII gene.

MSC, referred to in the field as mesenchymal stem cells, mesenchymal stromal cells, and, when isolated from bone marrow, also marrow stromal progenitors (MSP), are multipotent stromal cells that can differentiate into a variety of cell types, including: osteoblasts, chondrocytes, myocytes, and adipocytes. MSC do not have the capacity to reconstitute an entire organ. The term encompasses multipotent cells derived from other non-marrow tissues, such as placenta, umbilical cord blood, adipose tissue, or the dental pulp of deciduous baby teeth. MSC are heterogeneous and different subsets of MSC may have different capabilities. Different methods of isolation will result in different populations of MSC. Such different populations may express different protein markers. MSC subpopulations with different marker expression profiles have been found to have different capabilities. See, for example, Thierry, D., et al., Stro-1 Positive and Stro-1 Negative Human Mesenchymal Stem Cells Express Different Levels of Immunosuppression, Blood 104(11): 4964 (2004). The extent to which a MSC population isolated using one method and having a particular marker profile will share properties with a MSC population isolated using a different method and having a different marker profile has not been determined. In some instances, the MSC may be an established cell line that is publically available such as, for example, a commercial MSC cell line.

In some instances that do not form part of the claimed invention, the MSC used in the method are bone marrow-derived MSC - that is, the MSC are isolated from bone marrow. Specifically, the bone marrow-derived MSC are isolated from bone marrow obtained from an individual other than the subject on which the method is performed (allogenic MSC). In some instances, the bone marrow-derived MSC used in the method express Stro-1, CD146, or both Stro-1 and CD146. Flow cytometry methods may be used to isolate MSC expressing these markers such as described, for example, in Sanada C., et al., Mesenchymal stem cells contribute to endogenous FVIII:c production. J Cell Physiol. 2013; 228(5):1010-1016 and Chamberlain J.L., et al., Efficient generation of human hepatocytes by the intrahepatic delivery of clonal human mesenchymal stem cells in fetal sheep. Hepatology. 2007; 46(6):1935-1945. Isolating MSC based on Stro-1 and/or CD146 results in a distinct cell population from that isolated using the traditional approach in which bulk unpurified bone marrow or Ficoll-purified bone marrow mononuclear cells are plated directly into plastic cell culture plates or flasks to which the adherent MSC population binds.

The MSC used in the method are MSC isolated from a birth tissue or birth fluid. Specifically, the MSC are isolated from placental tissue. The MSC used in the method express c-kit. Methods of isolating such cells are described in U.S. Patent Nos 7,968,336 and 8,021,876. In some instances, the MSC express c-kit and at least one of CD34, CD90, or CD133. In some instances, the MSC are isolated based on expression of c-kit. If prenatal biological samples are available, the MSC may be isolated from such samples (placental tissue). MSC may be isolated from prenatal biological samples obtained prior to the subject's birth from the subject's mother. Alternatively, because the fetal environment is tolerogenic, MSC may be isolated from donated birth tissue biological samples, namely placental tissue.

The MSC used in the method are modified, via introduction of a transgene or genome editing, to express high levels of Factor VIII. In some instances, the MSC may be modified, via introduction of a transgene or genome editing, to also express high levels of von Willebrand factor (vWF).

In some instances, an exogenous gene sequence encoding one or both of these proteins may be introduced into the MSC via one or more vectors. In some instances, the MSC may be modified to express high levels of Factor VIII protein via introduction into the MSC of a vector comprising a Factor VIII gene sequence operatively linked to a constitutively active promoter. In some instances, the MSC may be modified to express high levels of vWF protein via introduction into the MSC of a vector comprising a vWF gene sequence operatively linked to a constitutively active promoter. In some instances, the MSC may be modified to express high levels of Factor VIII protein and vWF protein via introduction into the MSC of a vector comprising a Factor VIII gene sequence operatively linked to a constitutively active promoter and a vector comprising a vWF gene sequence operatively linked to a constitutively active promoter. In some instances, the Factor VIII gene sequence and the vWF gene sequence may be operatively linked to the same constitutively active promoter. Alternatively, the Factor VIII gene sequence and the vWF gene sequence may be operatively linked to different constitutively active promoters.

Exemplary vectors include, for example, plasmids and viral vectors (including but not limited to adenoviral, adeno-associated viral (AAV), or retroviruses such as lentiviruses. In preferred embodiments, the vector is a viral vector. In some instances, the vector may be a vector that integrates into the genome of transduced cells. For example, the vector may be a lentivirus vector. In preferred embodiments, the vector is a lentivirus vector. In some instances, the lentivirus vector contains a 3'-modified long terminal repeat (LTR), resulting in a self-inactivating (SIN) lentivector. A lentivirus vector may integrate into the genome of dividing or non-dividing cells. The lentivirus genome in the form of RNA is reverse-transcribed to DNA when the virus enters the cell, and is then inserted into the genome by the viral integrase enzyme. The lentivirus vector, now called a provirus, remains in the genome and is passed on to the progeny of the cell when it divides. In another example, the vector may be an adeno-associated virus (AAV) vector, which, in contrast to wild-type AAV, only rarely integrates into the genome of the cells it transduces. In one example, the vector may be an adenoviral vector. An adenoviral vector does not integrate into the genome. In another instance, the vector may be a murine retrovirus vector. In another example, the vector may be a foamy virus vector, which may have a larger capacity for inserts than lentiviral vectors. In another example, the vector may be Sendai virus vector.

The exogenous gene sequences are operatively linked to one or more promoter sequences within the vector. The term "promoter sequence" or "promoter element" refers to a nucleotide sequence that assists with controlling expression of a coding sequence. Generally, promoter elements are located 5' of the translation start site of a gene. However, in certain embodiments, a promoter element may be located within an intron sequence, or 3' of the coding sequence. In some embodiments, a promoter useful for a gene therapy vector is derived from the native gene of the target protein (e.g., a Factor VIII promoter). In some embodiments, the promoter is a constitutive promoter, which drives substantially constant expression of protein from the exogenous gene sequence. Non-limiting examples of well-characterized promoter elements include the cytomegalovirus immediate-early promoter (CMV), the β-actin promoter, the methyl CpG binding protein 2 (MeCP2) promoter, the simian virus 40 early (SV40) promoter, human Ubiquitin C promoter (UBC), human elongation factor 1α promoter (EF1α), the phosphoglycerate kinase 1 promoter (PGK), or the CMV immediate early enhancer/chicken beta actin (CAG) promoter. The vector will generally also contain one or more of a promoter regulatory region (e.g., one conferring constitutive expression), a transcription initiation start site, a ribosome binding site, an RNA processing signal, a transcription termination site, and/or a polyadenylation signal.

In some instances, the Factor VIII trangene is operably linked to a promoter. A number of promoters can be used in the practice of the invention. The promoters can be selected based on desired outcome. The nucleic acids can be combined with constitutive, inducible, tissue-preferred, or other promoters for expression in the organism of interest. See, for example, promoters set forth as SEQ ID NOs: 1-6 as described in Brown et al. (2018) Target-Cell-Directed Bioengineering Approaches for Gene Therapy of Hemophilia A. Mol. Ther. Methods Clin. Dev., 2018. 9:57-69.

Where the MSC are modified to express high levels of Factor VIII via transduction with an exogenous Factor VIII gene sequence, the exogenous Factor VIII gene sequence may be human Factor VIII gene sequence, porcine Factor VIII gene sequence, or a hybrid transgene comprising portions of human Factor VIII gene sequence and portions of porcine Factor VIII gene sequence. In some instances, the gene sequence comprises all or a portion of the human Factor cDNA as set forth in GenBank Accession No. 192448441 as updated July 17, 2017, wherein said portion would encode a function portion of the human Factor VIII protein. In some instances, the gene sequence comprises a sequence that is at least 99%, 98%, 97%, 96%, 95%, 94%, 93%, 92%, 90%, 89%, 88%, 87%, 86%, 85%, 84%, 83%, 82%, 81%, or 80% identical to all or a portion of the human Factor cDNA as set forth in GenBank Accession No. 192448441 as updated July 17, 2017, wherein said portion would encode a function portion of the human Factor VIII protein. In some instances, the gene sequence may encode all or a functional portion of the human Factor VIII protein as set forth in GenBank Accession No. 192448441 as updated July 17, 2017, reflecting the protein transcribed from transcript variant 1 of the Factor VIII gene. This protein is approximately 300 kDa and contains a series of homology-defined domains designated A1-A2-B-ap-A3-C₁-C₂. In some instances, the exogenous Factor VIII gene sequence is modified relative to wild-type protein sequence to result in increased protein expression, increased protein stability, reduced immunogenicity, or a combination of one or more thereof.

In some instances, the sequence of one or more of the Factor VIII protein domains may be deleted. In one example, the B domain of Factor VIII is deleted. The B domain of Factor VIII has no known function and can be deleted without loss of coagulant activity. Deletion of the B-domain has been shown to increase factor VIII protein production in heterologous systems (Toole et al. (1986) Proc. Natl. Acad. Sci. U.S.A. 83:5939-5942). In addition, wildtype porcine Factor VIII protein having the B-domain deleted may have 10-100-fold higher expression and secretion than the human Factor gene sequence, both *in vitro* and *in vivo.* (See, for example, Dooriss, K.L., et al., Comparison of factor VIII transgenes bioengineered for improved expression in gene therapy of hemophilia A. Hum Gene Ther. 20:465-478 (2009). A B-domain deleted form of human Factor VIII protein (Lind et al. (1995) Eur. J. Biochem. 232: 19-27) has been approved for clinical use.

In some instances, the exogenous Factor VIII gene sequence may include protein modifications to reduce immunogenicity of the protein thereby reducing the risk of an immune response due to therapy. For example, alanine substitutions may be included as described in Healey, J.F., et al., The comparative immunogenicity of human and porcine factor VIII in haemophilia A mice. Thromb Haemost. 102:35-41 (2009) and Lubin, I.M., et al., Analysis of the human factor VIII A2 inhibitor epitope by alanine scanning mutagenesis. J Biol Chem. 272:30191-30195 (1997).

In some instances, one or more of the human Factor VIII protein domain sequences may be substituted with the sequence of the corresponding porcine Factor VIII protein domain sequences. For example, one or more porcine Factor VIII domains may be substituted for one or more human Factor VIII domains. For example, inclusion of the porcine Factor VIII domains A1 and ap-A3 may increase expression of the expressed Factor VIII protein. See, for example, Doering, C.B., et al., Identification of porcine coagulation factor VIII domains responsible for high level expression via enhanced secretion. J Biol Chem. 279:6546-6552 (2004). In some embodiments, the exogenous Factor VIII gene sequence may comprise the human Factor VIII A2 and C2 domains and the porcine Factor VIII A1, A3, and C1 domains.

In some instances, the exogenous Factor VIII gene sequence may comprise a modified Factor VIII sequence comprising a B domain-deleted (BDD) Factor VIII transgene having the sequence of the human A2 and C2 domains and the porcine A1, A3, and C1 domains, and also include three alanine substitutions in the A2 domain to reduce immunogenicity, as described in Lubin et al., Analysis of the human factor VIII A2 inhibitor epitope by alanine scanning mutagenesis. J Biol Chem. 1997; 272(48):30191-5. This modified Factor VIII protein is referred to as the ET3 transgene in this disclosure, including in the Examples below. In some instances, the ET3 transgene is expressed at a comparable level to that of wild-type porcine Factor VIII protein while having 91% identity to the amino acid sequence of wild-type human Factor VIII protein. In one example, the exogenous Factor VIII gene sequence may comprise a human/porcine Factor VIII transgene as described in Doering et al., Directed engineering of a high-expression chimeric transgene as a strategy for gene therapy of hemophilia A, Mol. Ther. 17(7): 1145-1154 (2009).

In some instances, the Factor VIII transgene sequence may comprise one of the modified Factor VIII sequences described in Brown et al. (2018) Target-Cell-Directed Bioengineering Approaches for Gene Therapy of Hemophilia A. Mol. Ther. Methods Clin. Dev., 2018. 9:57-69. Factor VIII polypeptides, including tissue-specific codon optimized variants, are described therein. Modified Factor VIII trangene sequences used in the methods decribed herein can be any one of SEQ ID NOs: 7-16 (as described in Brown *et al*.). For example, Factor VIII trangene sequences that can be used in the methods described herein include a B-domain deleted (BDD) human Factor VIII polypeptide (HSQ) as set forth in SEQ ID NO: 15, a BDD chimeric human/procine Factor VIII polypeptide (ET3) as set forth in SEQ ID NO: 11, or an ancestral Factor VIII polyptide (An53) as set forth in SEQ ID NO: 7.

In some instances, the exogenous Factor VIII gene sequence may modified for expression in a particular organ or tissue type. For example, the gene sequence may be optimized for expression in myeloid tissue. In some embodiments, the Factor VIII transgene may comprise myeloid codon optimized ET3 (mcoET3) as set forth in SEQ ID NO: 12 or myeloid codon optimized HSQ (mcoHSQ) as set forth in SEQ ID NO: 16. Alternatively, the Factor VIII transgene may be optimized for expression in liver tissue. In some embodiments, the Factor VIII transgene may comprise liver codon optimized ET3 (lcoET3) as set forth in SEQ ID NO: 10; liver codon optimized An53 as set forth in SEQ ID NO: 8; or liver codon optimized (lcoHSQ) as set forth in SEQ ID NO: 14.

In some instances, the exogenous Factor VIII gene sequence may comprise one of the modified Factor VIII sequences described in U.S. Patent No. 7,635,763. Regions of the porcine Factor VIII polypeptide that comprises the A1 and ap-A3 regions, and variants and fragments thereof, are described therein that impart high-level expression to both the porcine and human Factor VIII polypeptide. The exogenous Factor VIII gene sequence encoded by the viral vector of the provided methods may be the polynucleotides set forth in any one of SEQ ID NOs: 19, 21, 23, 25, or 27 (SEQ ID NOs: 15, 17, 19, 13, or 21 as described in U.S. Patent No. 7,635,763). The modified Factor VIII protein expressed at high levels in the modified MSC may comprise the amino acid sequences set forth in any one of SEQ ID NOs: 18, 20, 22, 24, or 26 (SEQ ID NOs: 14, 16, 18, 12, or 20 as described in U.S. Patent No. 7,635,763). Such sequences are summarized in Table 1 below. In some instances, these sequences may be used to construct an exogenous Factor VIII gene sequence encoding a modified factor VIII polypeptide that results in a high level of expression of the encoded modified Factor VIII protein.

**Table 1. Exemplary Modified Factor VIII Proteins**

| Modified Factor VIII Protein | SEQ ID NO. | Description |
|---|---|---|
| HP44/OL | aa: SEQ ID NO:18 | A1_{P}-A2_{P}-ap_{P}-A3_{P}-C1_{H}-C2_{H} |
| | nt: SEQ ID NO: 19 | porcine A1, A2, ap-A3 domains, porcine-derived linker sequence S F A Q N S R P P S A S A P K P P V L R R H Q R (SEQ ID NO: 30), and human C1 and C2 domains |
| HP46/SQ | aa: SEQ ID NO: 20 | A1_{P}-A2_{H}-ap_{H}-A3_{H}-C1_{H}-C2_{H} |
| | nt: SEQ ID NO: 21 | porcine A1 domain, human A2, ap-A3, C1 and C2 domains, and human S F S Q N P P V L K R H Q R linker sequence |
| HP47/OL | aa: SEQ ID NO:22 | A1_{P}-A2_{H}-ap_{P}-A3ₚ-C1_{H}-C2_{H} |
| | nt: SEQ ID NO: 23 | porcine A1, ap-A3 domains, porcine-derived linker sequence S F A Q N S R P P S A S A P K P P V L R R H Q R (SEQ ID NO: 31), and human A2, C1 and C2 domains |
| B-domain deleted | aa: SEQ ID NO:24 | Human Factor VIII protein sequence minus B-domain |
| | nt: SEQ ID NO:25 | |
| HP63/OL | aa: SEQ ID NO:26 | porcine A1 domain and a partially |
| | nt: SEQ ID NO: 27 | humanized ap-A3 domain that comprises porcine amino acids from about 1690 to about 1804 and from about 1819 to about 2019 |

As discussed above, in some instances, the MSC are also modified to express high levels of vWF protein via introduction into the MSC of a vector. In some embodiments coding sequences for vWF can be any one of SEQ ID NOs: 28 or 29. In some instances, the vWF gene sequence in the vector may encode all or a functional portion of the human vWF protein set forth in GenBank Accession No. 1023301060 as updated August 21, 2017. However, in some instances, the vWF gene sequence may include one or more modifications to the wild-type vWF gene sequence to increase protein expression, increase protein stability, reduce immunogenicity, or a combination of one or more thereof, of the vWF protein. For example, the full cDNA sequence of the vWF gene may be too large to be packaged efficiently in certain vectors, such as, for example, a lentiviral vector. Thus, in some instances, one or more exons of the vWF gene may be deleted while still retaining biological function of the expressed protein. In some instances, exons 24-46 of the vWF gene may be deleted as described in U.S. Patent Application Publication No. 2010/0183556. In some instances, the vWF gene sequence may be codon-optimized for efficient expression in the MSC. In some instances, the exogenous vWF gene sequence may modified for expression in a particular organ or tissue type. For example, the gene sequence may be optimized for expression in the liver. Thus, in some instances, the vWF gene sequence may comprise at least 99%, 98%, 97%, 96%, 95%, 94%, 93%, 92%, 90%, 89% 88%, 87%, 86%, 85%, 84%, 83%, 82%, 81%, or 80% identity to the corresponding wild-type vWF gene sequence and comprise modifications to improve expression. In some instances, the vWF gene sequence comprises the truncated human vWF sequence set forth below in this disclosure or a sequence at least 99%, 98%, 97%, 96%, 95%, 94%, 93%, 92%, 90%, 89% 88%, 87%, 86%, 85%, 84%, 83%, 82%, 81%, or 80% identical thereto while retaining biological activity of the expressed protein. In some instances, the vWF gene sequence comprises the truncated sheep vWF sequence set forth below in this disclosure or a sequence at least 99%, 98%, 97%, 96%, 95%, 94%, 93%, 92%, 90%, 89% 88%, 87%, 86%, 85%, 84%, 83%, 82%, 81%, or 80% identical thereto while retaining biological activity of the expressed protein.

In some instances, gene-editing may be performed on the MSC to insert, delete, or replace the genomic sequence of one or both of the endogenous genes using engineered nucleases (molecular scissors). Gene-editing nucleases belong to one of three known categories: zinc-finger nucleases (ZFN), transcription activator-like effector nucleases (TALEN), and clustered regularly interspaced short palindromic repeats (CRISPR) and their associated proteins (Cas) tools. All operate on the same principle; they are all capable of inducing a double-strand break at a defined genomic sequence that is subsequently corrected by endogenous DNA repair mechanisms. Double-strand breaks can be repaired through homology-driven repair (HDR), in the presence of donor homologous DNA sequences, resulting in gene-editing events.

In some instances, the MSC may be modified to express high levels of the Factor VIII protein via gene-editing of an endogenous Factor VIII gene sequence of the MSC, wherein the gene editing introduces one or more modifications to an endogenous Factor VIII gene sequence that increase protein expression, increase protein stability, reduce immunogenicity, or a combination of one or more thereof, of the Factor VIII protein. In some instances, the MSC are modified to express high levels of an exogenous FVIII protein via genome-editing, wherein the gene editing introduces an exogenous FVIII gene, under the control of a constitutive promoter, into a "safe harbor" region within the genome, such as the AAVS1 site. In some instances, the MSC are modified to express high levels of the vWF protein via gene-editing of an endogenous vWF gene sequence of the MSC, wherein the gene editing introduces one or more modifications to the endogenous vWF gene sequence that increase protein expression, increase protein stability, reduce immunogenicity, or a combination of one or more thereof, of the vWF protein. In some instances, the MSC are modified to express high levels of an exogenous vWF protein via genome-editing, wherein the gene-editing introduces an exogenous vWF gene, under the control of a constitutive promoter, into a "safe harbor" within the genome, such as the AAVS1 site. Exemplary "safe harbor" regions are described in Cerbini, T., et al., Transfection, selection, and colony-picking of human induced pluripotent stem cells TALEN-targeted with a GFP gene into the AAVS1 safe harbor. J Vis Exp. 2015 Feb 1; (96):52504 and Hong, S.G., et al., Rhesus iPSC Safe Harbor Gene-Editing Platform for Stable Expression of Transgenes in Differentiated Cells of All Germ Layers. Mol Ther. 2017; 25(1):44-53.

In some instances, the endogenous Factor VIII gene sequence may be modified by gene-editing to have the type of modifications described above for embodiments where an exogenous Factor VIII gene sequence is introduced via transduction. The discussion of the various modifications described above is thus also applicable to embodiments where the endogenous Factor VIII gene sequence is modified. For example, in some instances, the sequence of one or more protein domains of the endogenous Factor VIII gene sequence may be deleted. In some instances, the B domain of Factor VIII is deleted. In some instances, the endogenous Factor VIII gene sequence may be modified to reduce immunogenicity of the protein thereby reducing the risk of an immune response due to therapy. For example, alanine substitutions may be introduced as described in Healey, J.F., et al., The comparative immunogenicity of human and porcine factor VIII in haemophilia A mice. Thromb Haemost. 102:35-41 (2009) and Lubin, I.M., et al., Analysis of the human factor VIII A2 inhibitor epitope by alanine scanning mutagenesis. J Biol Chem. 272:30191-30195 (1997).

In some instances, the endogenous Factor VIII gene sequence may be modified to substitute one or more of the Factor VIII protein domain sequences with the sequence of the corresponding Factor VIII protein domain sequences from another species. For example, for human subjects, the endogenous Factor VIII gene sequence may be modified to substitute one or more of the human Factor VIII protein domain sequences with the sequence of the corresponding porcine Factor VIII protein domain sequences. For example, substitution with the porcine Factor VIII domains A1 and ap-A3 may increase expression of the expressed Factor VIII protein. See, for example, Doering, C.B., et al., Identification of porcine coagulation factor VIII domains responsible for high level expression via enhanced secretion. J Biol Chem. 279:6546-6552 (2004). In some embodiments, the endogenous Factor VIII gene sequence may be modified to comprise the porcine Factor VIII A1, A3, and C1 domains, while retaining the human Factor VIII A2 and C2 domains.

In some instances, the endogenous Factor VIII gene sequence may be modified to include a B domain deletion, the porcine A1, A3, and C1 domains, and also include three alanine substitutions in the A2 domain to reduce immunogenicity, as described above for the exogenous Factor VIII gene sequence embodiments. In one example, the endogenous Factor VIII gene sequence may be modified to have the sequence of a human/porcine Factor VIII transgene as described in Doering, C.B., et al., Directed engineering of a high-expression chimeric transgene as a strategy for gene therapy of hemophilia A, Mol. Ther. 17(7): 1145-1154 (2009). In some instances, the modified endogenous Factor VIII gene sequence results in expression of a modified Factor VIII protein at a level comparable to that of wild-type porcine Factor VIII protein while having 91% identity to the amino acid sequence of wild-type human Factor VIII protein.

In some instances, the endogenous Factor VIII gene sequence may be modified to comprise one of the modified Factor VIII sequences described in U.S. Patent No. 7,635,763. In some instances, the endogenous Factor VIII gene sequence may comprise the polynucleotides set forth in any one of SEQ ID NOs: 19, 21, 23, 25, or 27 (SEQ ID NOs: 15, 17, 19, 13, or 21 as described in U.S. Patent No. 7,635,763). The modified Factor VIII protein expressed at high levels in the modified MSC may comprise the amino acid sequences set forth in any one of SEQ ID NOs: 18, 20, 22, 24, or 26 (SEQ ID NOs: 14, 16, 18, 12, or 20 as described in U.S. Patent No. 7,635,763). Such sequences are summarized in Table 1 above.

As discussed above, in some instances, the MSC are also modified to express high levels of vWF protein via gene-editing. In some instances, the vWF gene sequence may include one or more modifications to the wild-type vWF gene sequence to increase protein expression, increase protein stability, reduce immunogenicity, or a combination of one or more thereof, of the vWF protein. For example, in some instances, one or more exons of the vWF gene may be deleted while still retaining biological function of the expressed protein. In some instances, exons 24-46 of the vWF gene may be deleted as described in U.S. Patent Application Publication No. 2010/0183556. In some instances, the vWF gene sequence may be codon-optimized for efficient expression in the MSC. In some instances, the exogenous vWF gene sequence may modified for expression in a particular organ or tissue type. For example, the gene sequence may be optimized for expression in the liver. Thus, in some instances, the vWF gene sequence may be modified to comprise at least 99%, 98%, 97%, 96%, 95%, 94%, 93%, 92%, 90%, 89% 88%, 87%, 86%, 85%, 84%, 83%, 82%, 81%, or 80% identity to the corresponding wild-type vWF gene sequence and comprise modifications to improve expression. In some instances, the vWF gene sequence may be modified to comprise the truncated human vWF sequence set forth below in this disclosure or a sequence at least 99%, 98%, 97%, 96%, 95%, 94%, 93%, 92%, 90%, 89% 88%, 87%, 86%, 85%, 84%, 83%, 82%, 81%, or 80% identical thereto while retaining biological activity of the expressed protein. In some instances, the vWF gene sequence may be modified to comprise the truncated sheep vWF sequence set forth below in this disclosure or a sequence at least 99%, 98%, 97%, 96%, 95%, 94%, 93%, 92%, 90%, 89% 88%, 87%, 86%, 85%, 84%, 83%, 82%, 81%, or 80% identical thereto while retaining biological activity of the expressed protein.

Where the MSC are modified to express high levels of both Factor VIII and vWF, the same method of modification may be used to achieve high expression of both proteins or different methods could be used for each protein. For example, in some instances, the MSC may be modified to express high levels of both Factor VIII and vWF protein via introduction of exogenous gene sequences for both proteins. In another example, the MSC may be modified to express high levels of both Factor VIII and vWF protein via gene-editing of the endogenous gene sequences of both proteins. In some instances, the MSC may be modified to express high levels of Factor VIII via transduction of an exogenous Factor VIII gene sequence and modified to express high levels of vWF via gene-editing of the endogenous vWF gene sequences. In other instances, the MSC may be modified to express high levels of vWF via transduction of an exogenous vWF gene sequence and modified to express high levels of Factor VIII via gene-editing of the endogenous Factor VIII gene sequences.

A "high level of expression" means that the production/expression of the modified Factor VIII protein or vWF protein is at an increased level as compared to the expression level of the corresponding native Factor VIII protein or vWF protein expressed under the same conditions. An increase in protein expression levels (considered a high level of expression) comprises at least about 0.5, 1, 2, 3, 4, 5, 6, 7, 8, 9, 10, 11, 12, 13, 14, 15, 20-fold or greater expression of the modified Factor VIII protein or vWF protein compared to the expression levels of the corresponding Factor VIII protein or vWF protein. Alternatively, "high-level expression" can comprise an increase in protein expression levels of at least 1-25 fold, 1-5 fold, 5-10 fold, 10-15 fold, 15-20 fold, 20-25 fold or greater expression levels of the modified Factor VIII protein or vWF protein when compared to the corresponding Factor VIII protein or vWF protein. Methods for assaying protein expression levels are routine in the art. By "corresponding" Factor VIII protein or vWF protein is intended a Factor VIII protein or vWF protein that comprises an equivalent amino acid sequence. In one example, expression of a modified human Factor VIII protein comprising the A1-A2-ap-A3-C₁-C₂ domains is compared to a human Factor VIII protein containing corresponding domains A1-A2-ap-A3-C₁-C₂. In another example, for a fragment of a modified human Factor VIII protein containing domains A1-A2-ap-A3, expression is compared to a fragment of human Factor VIII protein having the corresponding domains A1-A2-ap-A3. Alternatively, in certain instances, expression of a modified Factor VIII protein or vWF protein may be compared to the full-length corresponding proteins. In one example, for a fragment of a modified human Factor VIII protein containing domains A1-A2-ap-A3, expression is compared to human Factor VIII protein having the A1-A2-ap-A3-C₁-C₂ domains.

The modified MSC are cultured *in vitro* to generate an expanded modified MSC population. The expanded modified MSC population provides sufficient numbers of modified MSC for therapeutic use. Culture conditions may be selected based on the type of MSC used in the method. MSC isolated from placental tissue may be grown in culture medium optimized for placental cells. In another example, which does not form part of the claimed invention, MSC isolated from amnion tissue may be grown in culture medium optimized for amniotic cells. In another example, which does not form part of the claimed invention, MSC isolated from umbilical cord or bone marrow may be grown in culture medium optimized for MSC cells. The modified cells may be grown on plastic culture dishes for at least 2, 3, 4, 5, or 6 passages to generate the expanded modified MSC population. In some instances, all or a portion of the expanded modified MSC population may be cryopreserved.

Following culturing of the modified MSC to generate expanded modified MSC population, modified MSC from expanded modified MSC population are injected into the subject in utero. The injection is preferably made via intraperitoneal injection. However, in some instances, the injection may be made into the umbilical cord vein. Each injection comprises about 10⁵ to about 10⁹ MSC from the expanded modified MSC population per kilogram weight of the subject. For example, the injection may comprise 10⁵ MSC, 10⁶MSC, 10⁷MSC, 10⁸ MSC, or 10⁹ MSC. The number of cells injected into the subject is based on the amount of protein expressed per cell. This metric is determined empirically for the expanded modified MSC population. In some instances, this metric may be generally predictable based on the nature of the modified MSC (for example, method of modification, Factor VIII gene sequence, vWF gene sequence, vector and vector components).

In some instances, modified MSC are injected into the subject once, twice, or 3 times. In some instances, modified MSC are injected into the subject at least once, at least twice, or at least 3 times. For example, the modified MSC may injected as multiple injections on the same day. In some instances, the modified MSC may be injected into the subject on multiple days. In some instances, the subject is injected with modified MSC on a first day and then the subject may be monitored after birth over a period of time (days or weeks) to determine if there is sufficient protein expression to provide the desired therapeutic benefit. Where monitoring indicates that the amount of expression of Factor VIII protein alone, or the amount of expression of Factor VIII protein and vWF protein, is insufficient, the subject's disease symptoms are not alleviated, or both, the postnatal subject may be injected with modified MSC on a second day. Again, the subject may be monitored over a period of time to determine if there is sufficient protein expression to provide the desired therapeutic benefit. These steps may be repeated for a fourth, fifth, sixth, seventh, eighth, ninth, or tenth injection after birth, as needed to achieve the desired therapeutic benefit of alleviating the subject's disease symptoms.

In some instances, the use of MSC as cellular vehicles to deliver an Factor VIII gene sequence, an vWF gene sequence, or both, to a subject (as opposed to administration of vector directly) may overcome limitations/risks observed to-date in AAV-based clinical trials for hemophilia: 1) the possibility of off-target transduction of troubling cell types, such as germline cells; 2) the inability to treat patients with pre-existing antibodies to the serotype of AAV being employed as a vector; and 3) the transient hepatotoxicity induced by the AAV capsid, that triggers subsequent immune/inflammatory destruction of many of the transduced cells. Although early studies *in vitro* and in normal and hemophilia A mice, have used unselected stromal cells (isolated based solely upon plastic adherence) as cellular vehicles for delivering exogenous Factor VIII, no attempts have yet been made to use phenotypically-defined MSC/pericytes to deliver FVIII in vivo in any preclinical model of hemophilia A.

In some instances, the use of MSC as cellular vehicles to deliver a therapeutic gene is also an improvement over the use of hematopoietic stem cells (HSC), as have been used in most cell-based gene therapy trials. The use of MSC eliminates the possibility of insertional leukemogenesis, which is the most serious adverse event seen to-date in clinical gene therapy trials. A successful outcome of the proposed studies targeting hemophilia A thus promises to open the door to safe correction of a variety of congenital disorders using MSC to deliver the therapeutic gene.

### EXAMPLES

Any examples not falling without the scope of the claim are provided for comparison purposes only.

### Example 1. Animal Model

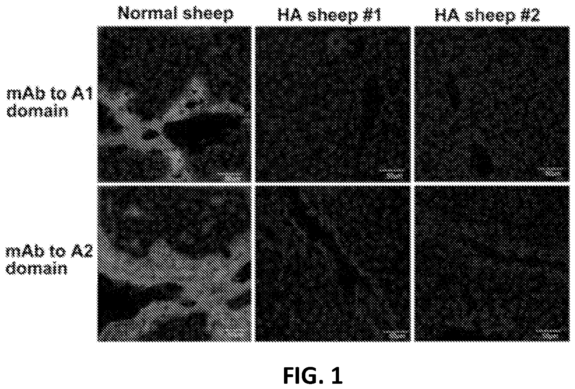

Applicant re-established a line of sheep that emulates the genetics, inhibitor formation, and clinical symptoms of the severe form of human hemophilia A (HA), including the development of frequent, spontaneous hematomas and crippling hemarthroses, making them unique among the HA models. See Porada, C.D., et al., Clinical and molecular characterization of a re-established line of sheep exhibiting hemophilia A. J Thromb Haemost, 2010. 8(2): 276-285. Using unique antibodies developed to various regions of the ovine FVIII protein, it was determined that these sheep do not produce any FVIII antigen/cross-reactive material (CRM) **(****FIG. 1****),** as demonstrated by the complete lack of any staining within the liver of two of the HA sheep, which is in marked contrast to the widespread bright staining that is seen in the liver from a normal/healthy sheep. As such, they should be an excellent model of severe, cross-reacting material (CRM)-negative hemophilia A patients. Additionally, sheep are close in size to humans, their immune system is quite similar to that of humans, and their long lifespan allows long-term efficacy and safety to be addressed.

### Example 2. Assess Carrier Cells for In Utero Therapy

Curative levels of FVIII expression can be obtained following intrauterine transplantation (IUTx) of cells engineered to stably express high levels of Factor VIII (FVIII), and achieving this goal will require both a cell that possesses optimal FVIII expression/secretion, and one that exhibits the appropriate levels and pattern of engraftment post-IUTx to ensure therapeutic levels of FVIII reach the circulation.

IUTx of transduced marrow stromal progenitors (*MSPs*) (bone marrow-derived MSC) or amniotic fluid progenitors (AFPs) (amniotic fluid-derived MSC) yields significant levels of durable, widespread engraftment and persistent expression of transgene products. We have recently shown that the level of endogenous FVIII expression by *MSPs* is very low (see Sanada C., et al., Mesenchymal stem cells contribute to endogenous FVIII:c production. J Cell Physiol. 2013; 228(5): 1010-1016). Thus, it is unlikely that IUTx of unmodified cells could fully correct HA.

The aim of this study is to delineate, in normal fetal recipients, the engraftment pattern and levels of circulating FVIII achieved following IUTx with FVIII-transduced MSPs, AFPs, or placenta-derived MSC (PLC) to determine if, despite the immune immaturity of the fetal recipient, autologous cells will engraft and persist at higher levels than either haploidentical (maternal) or allogeneic sources. It is possible that the fetus is not as immuno-naive as once presumed. Studies in mice have shown that IUTx with syngeneic cells results in higher levels of engraftment than when allogeneic cells with an identical phenotype are transplanted, and have also provided evidence that the maternal immune system may act as a barrier to engraftment following IUTx. Thus, maternal cells may be a better source of donor cells than paternal cells, which have been used in most clinical IUTx studies to-date. Also, in the sheep model described in Example 1, we have shown that IUTx with MSPs modified with vectors to over-express HLA-G or proteins cytomegalovirus uses for immune evasion results in significantly higher engraftment levels, even early in gestation. Thus, it is possible that engraftment could be enhanced by using autologous or haploidentical maternal cells. We will, therefore, directly compare the levels of engraftment and FVIII expression that are obtained with *autologous, haploidentical* (maternal), and *allogeneic* cells in this large-animal, preclinical model to determine the optimal cellular vehicle for treating HA in utero.

The ultimate goal is to use human cells to perform definitive clinical studies, human cells are inappropriate for these mechanistic studies, since they would not allow us to address the critical issue of whether *autologous* or maternal (*haploidentical*) cells durably engraft at higher levels than *allogeneic* cells. For this reason, we will use sheep MSPs and AFPs throughout these studies.

MSPs will be isolated from the bone marrow (BM) of the ewe carrying each fetus to be treated *(haploidentical),* or from an age-matched unrelated ewe (*allogeneic*), using methodology we have previously employed. We have elected to use females as donors for *allogeneic* MSPs to allow a direct and meaningful comparison to be made to *haploidentical* (maternal) MSPs without any possible gender effects confounding the data. The methods to be employed reliably yield primary sheep MSPs that are phenotypically and functionally similar to their human counterparts. Flow cytometry demonstrated these sheep MSPs are devoid of hematopoietic cells, as evidenced by the lack of expression of CD1 1b, CD34, and CD45, but express CD146 and CD90, markers that have been used to identify MSPs in other species. Anti-ovine antibodies to other CD antigens routinely used to identify MSPs, such as CD44, CD105, and CD73, are not currently available. Immunofluorescence microscopy demonstrated expression of vimentin and α-SMA, known MSP cytoskeleton proteins. We also verified their ability to differentiate into adipocytes (Oil-Red-O staining), osteocytes (Ca2+ deposits and alkaline phosphatase), and chondrocytes (Alcian blue, not shown). To ensure reproducibility and robustness of data, we will confirm the phenotype and *in vitro* differentiative capacity of an aliquot of each MSP population isolated throughout these studies, and all MSPs will be used at passage 2-4.

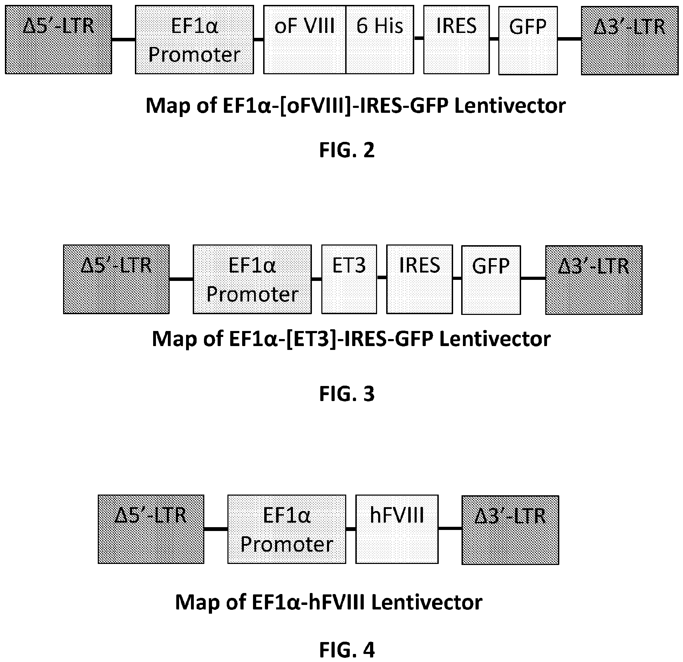

To obtain *autologous* and *allogeneic* AFPs, we will collect amniotic fluid at 35-45 gestational days and isolate/propagate AFPs using a method that consistently yields a homogeneous population of highly proliferative, multipotent AFPs (see Shaw, S.W., et al., Autologous transplantation of amniotic fluid-derived mesenchymal stem cells into sheep fetuses. Cell Transplant, 2011. 20(7):1015-31). Autologous or allogeneic placental MSC will be obtained by chorionic villus sampling. We will then perform 2 rounds of transduction with the EF1α-[oFVIII]-IRES-GFP lentivector **(****FIG. 2****)** at MOI: 5-50. See Lin, P., et al., Efficient lentiviral transduction of human mesenchymal stem cells that preserves proliferation and differentiation capabilities. Stem Cells Transl Med, 2012. 1(12):886-97, McGinley, L., et al., Lentiviral vector mediated modification of mesenchymal stem cells & enhanced survival in an in vitro model of ischaemia. Stem Cell Res Ther, 2011. 2(2):12. We have cloned and expressed both full-length and B-domain deleted recombinant ovine FVIII (oFVIII) as described in Zakas, P.M., et al., Development and characterization of recombinant ovine coagulation factor VIII. PLoS One, 2012. 7(11):e49481. The EF1α-[oFVIII]-IRES-GFP lentivector contains a B-domain deleted oFVIII gene having the polynucleotide sequence set forth in SEQ ID NO: 33. Although oFVIII is not a very high-expressing FVIII variant compared to FVIII from other species, the consensus in the HA field is that using "same-species" FVIII is essential in preclinical studies to accurately model the clinical response in patients. This vector system transduces >70% of human MSPs without altering their proliferation or functionality. All lentivectors use the constitutive EF1α promoter to drive FVIII, and contain the 3'-modified LTR to produce SIN lentivectors. Using a SIN vector and the EF1α promoter, which lacks an enhancer and cannot transactivate cellular genes, further safeguards against the risk of insertional mutagenesis, and ensures long-term stable FVIII expression in all progeny of the transplanted MSPs and AFPs, regardless of their phenotypic fate. We designed the lentivector encoding oFVIII to add a "6-His" tag (SEQ ID NO: 32) to the C-terminus of oFVIII. The inclusion of this tag will enable us to perform a straightforward ELISA to the 6-His tag (SEQ ID NO: 32) to reliably measure the levels of vector-driven oFVIII within the plasma of recipients that possess normal levels of endogenous oFVIII. This approach will allow us to use normal/healthy fetal sheep recipients for all of the initial studies to assess the effects of donor cell source and cell type on engraftment levels/distribution.

As this vector also encodes GFP (which is not immunogenic in utero and is expressed long-term in sheep, mice, rats, and nonhuman primates following in utero delivery), we will sort the GFP+ cells at 72 hr post-transduction using FACS to ensure nearly all cells are transduced prior to transplant. Following sorting, the GFP+ cells will then be transplanted into normal fetal sheep at 55-65 days of gestation, using a clinically relevant dose of 10⁸ cells/kg. An aliquot of each transplanted cell type will be reserved to determine vector copy number by qPCR and to perform integration site analysis by ligation-mediated PCR. Four transplantation groups will be included: 1) transduced autologous AFPs alone (n=5-6 fetuses); 2) transduced allogeneic AFPs alone (n=5-6 fetuses); 3) transduced maternal (haploidentical) MSPs alone (n=5-6 fetuses); and 4) transduced allogeneic MSPs alone. A group of normal/healthy, untransplanted animals (n=5-6 fetuses) will also be included as a control/reference for all proposed experiments.

Five to six fetuses will be used per experimental group to ensure differences observed in engraftment and/or circulating FVIII levels are statistically significant and reproducible. This number was determined by power analyses using the G*Power v3.1.9.2 software program to achieve significance, assuming a type I error of 5%, an effect size of 5 (percentage of normal FVIII levels needed to convert severe HA to mild), a standard deviation in engraftment of 3% within each group (based on our prior studies and a desired power of 80%). This conclusion was then verified using the "resource equation method", which predicts that an "E" value of >20 is achieved with 5 animals/group, confirming no additional statistical value would be obtained with increased group size. As HA is an X-linked disease, only HA males will be evaluated for phenotypic correction and tolerance induction. Preliminary data suggest that levels of engraftment after IUTx is not gender-related, but female and male fetuses will be used in the studies with normal recipients.

At 60 days post-IUTx, 3 sheep/group will be euthanized and major organs harvested and sectioned. Five to ten slides/tissue will be analyzed/quantitated for donor (GFP+) cells and for the 6-His tag present on the vector-encoded oFVIII, to determine the percentage and localization (parenchymal vs. perivascular) of engrafted cells that are expressing FVIII and could therefore provide therapeutic benefit for HA. Both visual examination (investigator will be blinded to sample identity to avoid bias) and Image-Pro software (Media Cybernetics) will be used for analysis/quantitation. We will also collect plasma from each recipient and quantitate circulating vector-derived FVIII levels using a 6-His ELISA, correlating engraftment levels/patterns with circulating FVIII levels.

While confocal analysis provides a fairly accurate estimate of the levels of GFP+ donor cells within each tissue, the slides selected for quantitation may or may not be representative of the engraftment levels within the organ as a whole. Therefore, we will also use a commercial ELISA (Cell Biolabs) that quantitates GFP within tissue homogenates with exquisite specificity and sensitivity. We will create a standard curve with known numbers of GFP+ *MSPs* and/or *AFPs* to establish how much GFP is present on a per-cell basis. Protein extracts from each tissue will then analyzed using this ELISA, comparing the tissue values to that of the standard curve, to precisely quantitate the number of MSPs/AFPs that have engrafted within each tissue. We will then compare the levels of donor MSPs/AFPs in each tissue with the resultant plasma FVIII levels to determine in which tissues engraftment produces the highest circulating levels of FVIII. To ensure the data generated are robust, tissues/tissue sections from the age-matched untransplanted animals will be used as controls in all confocal and ELISA experiments. In addition, all ELISA samples will be de-identified and run in triplicate to avoid any possibility of experimenter bias and ensure reproducibility of the data, respectively.

The remaining 2-3 sheep/group will be used for long-term analysis of plasma FVIII (by 6-His ELISA), to determine both the levels and duration of vector-encoded FVIII expression after IUTx of transduced autologous or allogeneic AFPs, haploidentical (maternal) MSPs, and allogeneic MSPs, and to determine whether combining AFPs with MSPs (at the same total cell dose) enhances circulating FVIII levels, due to the differing levels and/or patterns of engraftment of these two cell types. At euthanasia, all tissues will be examined to confirm that the long-term engraftment of MSPs or AFPs constitutively expressing high FVIII levels produces no untoward effects.

Collectively, these experiments will define the levels and pattern of engraftment provided by each cell type, and enable us to correlate the levels of FVIII-expressing donor cells within each tissue to plasma FVIII levels. These data will allow us to determine the optimal cell source(s) for delivering a FVIII transgene and guide us as to the levels and pattern of engraftment that will likely be therapeutic in HA animals.

### Example 3. Optimizing Factor VIII Expression in MSC For In Utero Transplantation

While we hypothesize that *autologous* AFPs (amniotic fluid-derived MSC) and placental MSC (pMSC) will be highly effective for treating HA by IUTx, it may not always be feasible/possible to collect and use *autologous* AFPs and pMSC clinically. Moreover, the ability to utilize maternal (*haploidentical*) or *allogeneic* (off-the-shelf) cells would eliminate any minimal risk associated with collecting amniotic fluid, and would greatly facilitate the clinical implementation of IUTx for HA. Diagnosis of HA and other genetic disorders can reliably be made as early as 7 weeks, by analyzing cell-free fetal DNA present in maternal blood. However, amniocentesis can only safely be performed beginning at about 16 gestational weeks. The ability to use maternal (*haploidentical*) MSPs or *allogeneic* (off-the-shelf) MSPs (or AFPs or pMSC) would thus make it possible to perform IUTx much sooner following diagnosis. For this clinically important reason, we will perform studies to boost FVIII expression/secretion in MSPs to allow their use/testing in the *in vivo* studies in HA animals as described in Example 8.

Human FVIII is produced at levels 3 orders of magnitude lower than other similarly sized, secreted glycoproteins, both *in vivo* and *in vitro.* The primary determinants of this biosynthetic limitation are specific amino acid sequences within the A1 and A3 domains of the molecule itself that enable FVIII to interact with multiple chaperones during its post-translational processing and trafficking from the endoplasmic reticulum (ER) to the Golgi. Chaperones such as BiP/Grp78 and phytanoyl-CoA α-hydroxylase (PAHX/PHYH) promote retention of nascent FVIII in the ER, leading to its degradation, which greatly reduces the amount of FVIII secreted from the cell. In recent studies, we showed that MSC from bone marrow, lung, liver, and brain all endogenously express FVIII mRNA and functional protein, but the levels of FVIII expression vary markedly in MSC from these 3 tissues, with MSPs producing nearly an order of magnitude less FVIII than MSC from the other tissues. See Sanada C., et al., Mesenchymal stem cells contribute to endogenous FVIII:c production. J Cell Physiol. 2013; 228(5):1010-1016. In preliminary microarray studies, we found that MSPs express very high levels of both BiP/Grp78 and PAHX/PHYH, which agrees with published transcriptome/proteome data on these cells (data not shown). In contrast, MSC from the other tissues expressed these chaperones at 1-2 orders of magnitude lower levels, providing a mechanistic explanation for the low inherent FVIII-producing capacity of MSPs. This is important, as low inherent FVIII-production/secretion cannot simply be circumvented via standard transgene expression technologies such as more efficient promoters/enhancers, transgene copy number, and codon optimization.

To test the hypothesis that altering binding of FVIII to the ER/Golgi chaperones BiP/Grp78 and PAHX/PHYH will enhance FVIII production/secretion in MSPs, we will make use of the ET3 bioengineered Factor VIII transgene as described in this disclosure (SEQ ID NO: 11). ET3 includes deletion of the B domain and substitution of a limited number of amino acids from "high expression" A1 and ap-A3 domains of recombinant porcine (rp)FVIII sequences into recombinant human FVIII (rhFVIII). These "high expression" sequences diminish interactions with the ER chaperones BiP/Grp78 and PAHX/PHYH, and attenuate induction of the unfolded protein response, resulting in 10- to 100-fold improved biosynthesis over FVIII constructs from other species (including human and sheep). Despite accounting for only 9% overall sequence modification, these substitutions have been shown to be necessary and sufficient to confer 10- to 100-fold enhancement in biosynthesis in a variety of cells from multiple species.

To test the ability of the bioengineered ET3 FVIII transgene (SEQ ID NO: 11) to overcome the limited FVIII production/secretion in MSPs and make them a viable therapeutic alternate to AFPs, we will perform a rigorous, head-to-head comparison between these cell types. We will isolate MSPs, as detailed in Example 2, and transduce aliquots of these cells in parallel with the EF1α-[oFVIII]-IRES-GFP SIN lentivector **(****FIG. 2****)** or with the EF1α-[ET3]-IRES-GFP SIN lentivector **(****FIG. 3****).** The EF1α-[oFVIII]-IRES-GFP SIN lentivector contains an oFVIII sequence having the polynucleotide sequence set forth in SEQ ID NO: 33. The EF1α-[ET3]-IRESGFP SIN lentivector contains an ET3 sequence having the polynucleotide sequence set forth in SEQ ID NO: 11. Sheep AFPs transduced with the EF1α-[oFVIII]-IRES-GFP SIN lentivector will be included to allow direct comparison with MSPs. We will sort cells at 72 hours post-transduction using FACS, to obtain populations in which nearly identical levels of cells are transduced and expressing the transgene, thus allowing for accurate comparisons to be made. Following FACS-sorting, cells will be plated and grown to approximately 70% confluence and then switched to serum-free media. Supernatants will then be collected 24 hr and 48 hr later and assayed to measure the levels of FVIII protein (ELISA) and activity (aPTT and chromogenic assay). Cells will also be harvested at this time, and DNA and RNA isolated therefrom. DNA will be used to perform qPCR to quantitate the average vector copy number present within each of the transduced cell populations, and the RNA will be used to precisely determine the levels of FVIII mRNA present within each cell population by qRT-PCR. FVIII activity in each population will be compared to quantify the levels of FVIII protein/activity per integrated provirus and per mRNA molecule in each cell type. These studies will provide a clear-cut answer whether FVIII production/secretion by *MSPs* can be boosted sufficiently to enable their use in IUTx to treat HA. All experiments will be repeated >3 times, using cells isolated from different donors.

### Example 4. Transduction Efficiency with Different Vectors

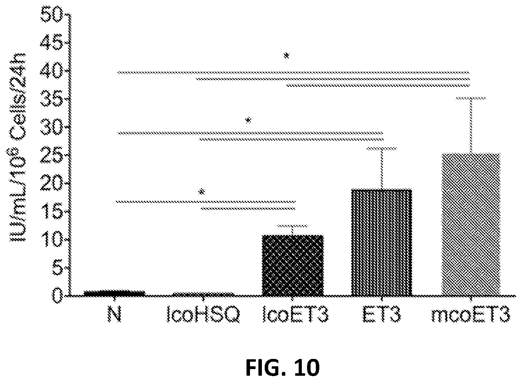

The transduction efficiency, FVIII production, and FVIII secretion from human PLC were compared following transduction at an identical multiplicity of infection (MOI) of 7.5 with an identical lentiviral vector (LV) encoding one of the following four different FVIII transgenes: (1) a bioengineered human-porcine hybrid FVIII (ET3) having the polynucleotide sequence set forth in SEQ ID NO: 11; (2) a liver codon-optimized ET3 (lcoET3) having the polynucleotide sequence set forth in SEQ ID NO: 10; (3) a liver codon-optimized human FVIII (lcoHSQ) having the polynucleotide sequence set forth in SEQ ID NO: 14; and (4) a myeloid-codon optimized ET3 (mcoET3) having the polynucleotide sequence set forth in SEQ ID NO: 12. Brown et al. (2018) Mol. Ther. Methods Clin. Dev. 9:57-69, demonstrated that vectors encoding FVIII, when codon-optimized to the target cells, or tissue, result in a dramatically increase FVIII expression of functional FVIII. Following transduction, PLCs were analyzed by flow cytometry and confocal microscopy to measure transduction efficiency and FVIII production. Conditioned media of PLCs were assayed by aPTT to quantitate FVIII activity. Analysis of the culture supernatants by aPTT demonstrated FVIII activity was readily detectable in supernatants of all transduced cells lines. It also revealed marked differences in the secretion of functional FVIII following transduction with each of these vectors. Specifically, PLCs transduced with mcoET3 (SEQ ID NO: 12), ET3 (SEQ ID NO: 11), lcoET3 (SEQ ID NO: 10), and lcoHSQ (SEQ ID NO: 14) LV secreted 25±9, 19±8, 11±2, and 1±0.1 IU of FVIII/24h/10⁶ cells, respectively **(****FIG. 10****).** PLC population doubling time was not affected by transduction with any of the vectors; nor were phenotype or expression of signaling molecules involved in innate immunity. Importantly, at passage 3 following transduction with any of the 4 lentiviral vectors, PLCs continued to produce and secrete FVIII at similar levels to those observed shortly after transduction, demonstrating stable vector integration and durability/longevity of FVIII expression. The relative levels of FVIII expression by PLCs following transduction with each lentiviral vector were also assessed by immunofluorescence microscopy with an antibody specific to a region of FVIII that is conserved in all 4 FVIII transgenes. These analyses confirmed the results of the aPTT analyses on the supernatants from these cells, with mcoET3-PLC exhibiting the brightest/highest intensity staining for FVIII, followed by PLC transduced with ET3 (SEQ ID NO: 11), then those transduced with lcoET3 (SEQ ID NO: 10) and with lcoHSQ (SEQ ID NO: 14) (data not shown).

The gene transfer efficiency of these gene-modified cells was assessed by determining the final proviral/vector copy number (VCN) using a commercially available qPCR-based kit (Lenti-X Provirus Quantitation Kit, Takara Bio USA, Inc., Mountain View, CA). To ensure that only integrated copies were detected by the assay, qPCR for VCN was performed in PLCs that had been passaged at least three times after transduction. After transducing the cells at the same MOI (7.5) with each lentiviral vector, the VCNs for mcoET3-PLC, lcoHSQ-PLC, lcoET3-PLC, and ET3-PLC were all around 1.

### Example 5. Optimizing Factor VIII Expression in Placental Cells

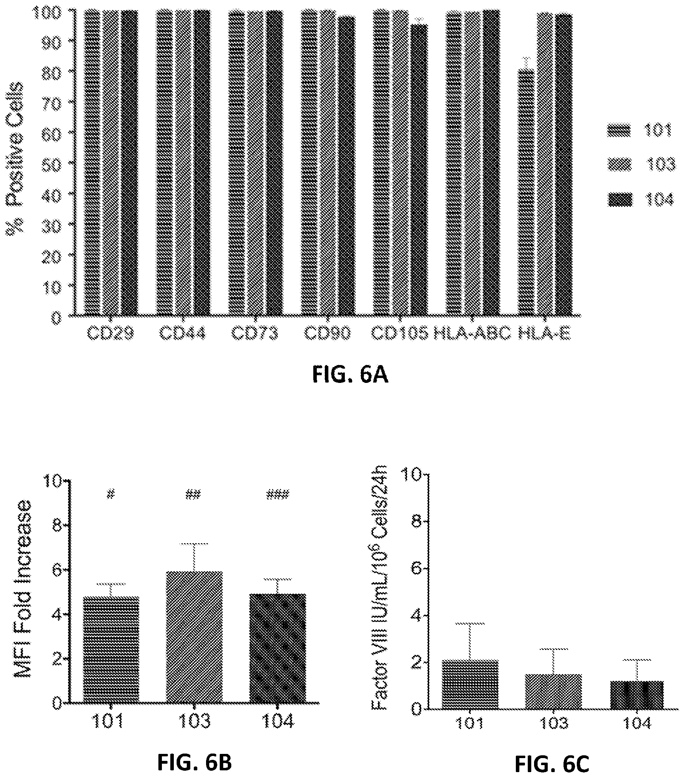

The aim of this study was to investigate the suitability of placental cells (PLC) as cellular delivery vehicles for FVIII. The expression of phenotypical markers was determined in three different master cells banks (101, 103, and 104) of placental cells (PLCs), each of which was derived from a different human donor by the Regenerative Medicine Clinical Core (RMCC) at WFIRM following GMP-compliant standard operating procedures (SOPs) established by the RMCC for PLC. Expression of CD29, CD44, CD73, CD90, CD105, HLA-ABC, HLA-E, CD31, CD34, CD35, CD144, HLA-G, HLA-DR/DP/DQ, and ABO blood group were determined using flow cytometric analysis. These markers were selected to confirm that the PLC isolated possessed a phenotype characteristic of MSC from other tissues (CD29, CD44, CD73, CD90, CD105), to assess their potential for stimulating an immune response upon transplantation (HLA-ABC, HLA-E, CD35, CD144, HLA-G, HLA-DR/DP/DQ, and ABO blood group), and to discern whether they expressed markers indicative of endothelial properties (CD31, CD34)._No statistically significant differences (p<0.05) were found in expression of phenotypic markers between PLCs derived from three different master cell banks (101, 103, and 104). PLCs from each of the master cell banks expressed CD29, CD44, CD73, CD90, CD 105, HLA-ABC, and HLA-E **(****FIG. 6A****);** had negligible amounts (<1%) of CD31, CD34, CD35, CD144, HLA-G, and HLA-DR/DP/DQ (data not shown); and were devoid of ABO blood group (data not shown). Collectively, these findings support the conclusion that PLC are an MSC-like population and that they should exhibit minimal immunogenicity upon transplantation.

PLCs derived from each of the master cell banks [MCBs] (101, 103, and 104) were assessed for their ability to express FVIII protein constitutively. Immunofluorescence microscopy with a primary antibody specific to hFVIIIc and a fluorochrome-conjugated secondary antibody and flow cytometric analysis with fluorochrome-conjugated antibodies to were used to determine the levels of constitutively expressed FVIII protein and define the phenptype of the PLCs, respectively. As shown in **FIG. 6A****,** these cells expressed markers characteristic of MSC from bone marrow and other tissues. All three MCBs endogenously expressed detectable amounts of FVIII by immunofluorescence microscopy, and MCB 103 expressed the highest levels, as indicated by the brightest fluorescence intensity (data not shown). The fold increase of FVIII expression over isotype control for PLCs derived from each of the MCBs is presented as relative mean fluorescence intensity (MFI) as shown in **FIG. 6B****.**

The activated partial thromboplastin time (*aPTT* or PTT) assay is a functional measure of the intrinsic and common pathways of the coagulation cascade (i.e. it characterizes blood coagulation). The aPTT assay was used to quantitate levels of functional FVIII secreted by PLCs. PLCs were plated at the same density and cultured for 24 hours in Phenol Red-free alpha-MEM AmnioMax Complete Medium (ThermoFisher Scientific, Raleigh, NC). Supernatants were collected and the number of cells present counted, and then the levels of secreted functional FVIII were measured by the Clinical Hematology Laboratory at Wake Forest Baptist Health using a commercial aPTT assay. Levels of FVIII were normalized by adjusting to account for the number of cells present at the time of supernatant collection, and expressing FVIII activity on a per cell basis. The data from this analysis is shown in **FIG. 6C**

FVIII mRNA levels in the PLCs derived from three different master cell banks (101, 103, and 104) were evaluated by qPCR using primers specific to human FVIII. Relative expression of endogenous mRNA for FVIII was calculated by comparing the threshold cycle (CT) value for FVIII with the CT of each master cell bank's respective internal reference gene, GAPDH. The relative expression of endogenous FVIII mRNA was 0.01±0.0005, 0.075±0.007, and 0.011±0.0002, for PLCs 101, 103, and 104, respectively.

### Example 6. Suitability of PLCs as a transgenic FVIII production platform

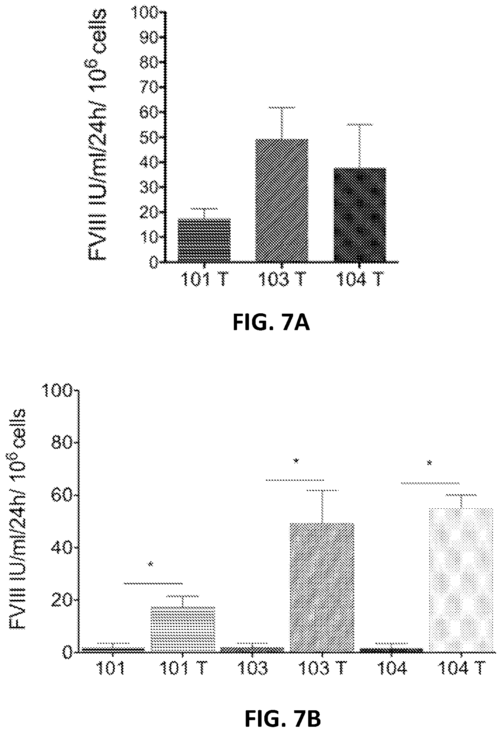

PLC 101, 103, and 104 were transduced at the same MOI (7.5) using a lentiviral vector-(LV) encoding mcoET3 (mcoET3-PLC) as described in Example 4 above. Vector copy number (VCN) was determined, as described in detail above. The VCN was found to be similar between the three different PLC MCBs (0.71-0.75). After transduction, the relative levels of expression of FVIII by the 3 MCB PLCs were assessed by immunofluorescence microscopy after staining with a primary antibody specific to hFVIIIc and a fluorochrome-conjugated secondary antibody. All 3 MCBs expressed high levels of FVIII after transduction with the mcoET3 lentiviral vector, but MCB 103 exhibited the highest levels of FVIII protein, as evidence by the brightest/highest fluorescence intensity (data not shown). The secretion of FVIII was determined using aPTT performed on 24-hour culture supernatants harvested from PLCs that were plated at the same density and normalized for the number of cells present at the time of the supernatant collection, as described in detail in the preceding paragraphs **(****FIG. 7A****).** Levels of FVIII in the culture supernatant increased significantly (p<0.05) in PLCs derived from all 3 MCBs (101, 103, and 104) when compared with respective non-transduced PLC counterparts **(****FIG. 7B****).** No significant differences were found between the different transduced cells.

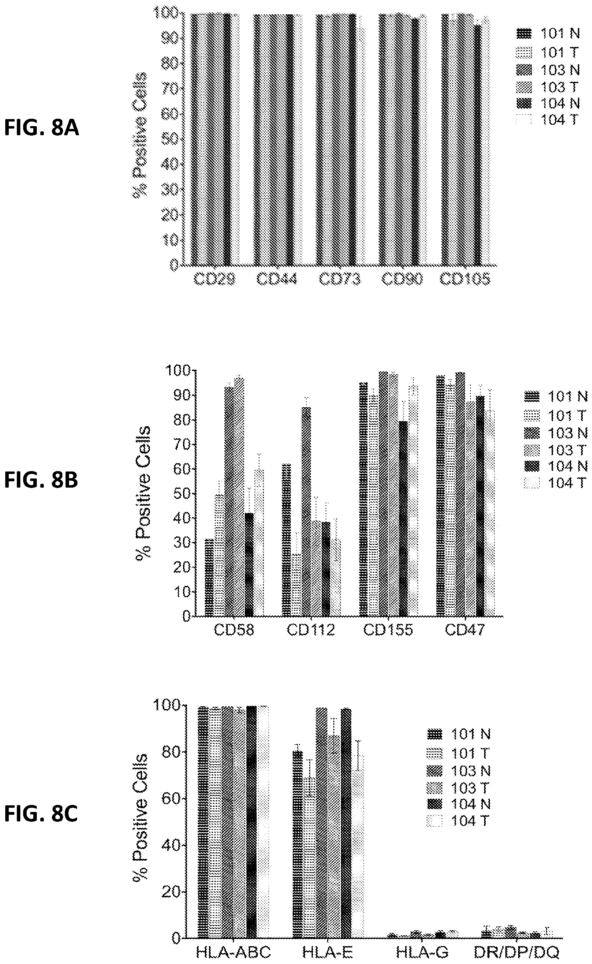

The effect of transduction of PLCs with LV encoding mcoET3 on phenotype and molecules involved in immunity was assessed. Expression of CD29, CD44, CD73, CD90, CD 105, CD58, CD112, CD155, CD47, HLA-ABC, HLA-E, HLA-G, and HLA-DR/DP/DQ, were determined by flow cytometric analysis, as described above. No statistically significant differences (p<0.05) were found between transduced and non-transduced cells **(****FIGS. 8A-8C****).** Additionally, PLC population doubling time was not affected by transduction (data not shown). Both non-transduced and transduced PLCs expressed CD47, a molecule involved in immune evasion **(****FIG. 8B****).** Transduced cells did not significantly upregulate the expression of HLA-DR/DP/DQ **(****FIG. 8C****).**

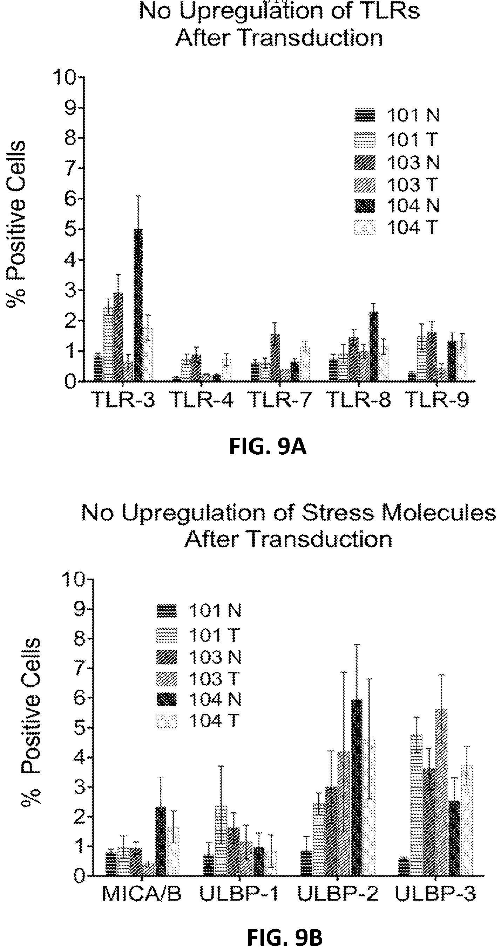

To further examine whether transduction of the PLC with the mcoET3 lentiviral vector had the potential to alter the immunogenicity of these cells, we examined the levels of expression of various Toll-like Receptors (TLRs) on the PLC prior to and following transduction, as these molecules play a key role in innate immunity, and their upregulation could potentially trigger an immune response to the transduced cells upon transplantation. To address this possibility, the effect of PLC transduction with the mcoET3 lentiviral vector on TLR-3, TLR-4, TLR-7, TLR-8, and TLR-9 expression was assessed. TLR expression on transduced (t) and non-transduced (n) PLCs (101, 103, and 104) was determined using flow cytometric analysis. No significant differences in expression of TLR molecules was detected in the PLC populations. As shown in **FIG. 9A****,** there was no difference in the levels of expression of any of these TLRs in tranduced vs. non-transduced PLCs, confirming that transduction of these 3 MCB PLCs with the mcoET3 lentiviral vector did not lead to upregulation of any of these immune-stimulating molecules.

In order to evaluate the demands of PLC transduction with mcoET3 and increased Factor VIII expression on the secretory and endoplasmic reticulum pathways, expression of stress molecules MICA/B, ULBP-1, ULBP-2, and ULBP-3 was determined in transduced (t) and non-transduced (n) PLCs (101, 103, and 104). Flow cytometric analysis demonstrated that no significant expression or alteration/upregulation of MICA/B or ULBP-1 were found before or after transduction with mco-ET3 lentiviral vector **(****FIG. 9B****).**

The production of interferon-gamma (IFN-γ) by mcoET3 transduced and non-transduced PLCs was measured using a high-sensitivity ELISA (assay range: 0.16-10.0 pg/mL). PLCs were cultured for 24 hours in AmnioMax Complete Medium (ThermoFisher). Supernatants were collected and IFN-γ production was determined. No IFN-γ was detected in any of the culture supernatants of mco-ET3 transduced or non-transduced PLCs (data not shown).

### Example 7. FVIII and vWF Transduction of PLCs

PLCs were isolated based on c-kit positivity under GMP conditions. Characterization of PLCs determined that PLCs (Passage 3-15) constitutively expressed vWF and constitutively produced and secreted functional FVIII at 0.4-0.5 IU/10⁶cells/24h. Thus, PLCs possess the requisite machinery to efficiently produce and process FVIII. PLCs were transduced with a lentivirus vector encoding mcoET3 (SEQ ID NO: 12)-; a lentivirus vector encoding human vWF (SEQ ID NO: 28); or both lentivirus vectors. Untransduced and transduced PLCs were stained with primary antibodies to FVIII and vWF, followed by fluorochrome-conjugated secondary antibodies specific to the species of the respective primary antibody, and were then assessed using immunofluorescence microscopy. Human umbilical vein endothelial cells (HUVECs) were used as a positive control for vWF and human hepatic sinusoidal endothelial cells (HHSEC) were used as a positive control for FVIII. Untransduced human PLCs endogenously produced low, but readily detectable levels of both FVIII and vWF (data not shown). As demonstrated by the increase in fluorescence intensity, transduction with mcoET3-encoding LV increased production and secretion of FVIII by PLCs and also increased the endogenous expression of vWF.

At passage 3 after transduction with a LV encoding a bioengineered and codon-optimized FVIII transgene, designated mcoET3, under the control of the constitutive EF1α promoter, PLCs stably secreted 27.9 IU FVIII/106 cells/24h (data not shown). To assess the efficiency of transduction and the relative safety of these gene-modified cells, qPCR was performed with vector-specific primers, and amplification compared to that obtained with primers to a known single-copy gene. These analyses yielded a vector copy number of 0.35±0.05 per diploid genome equivalent (data not shown).

### Example 8. In Utero transplantation of mcoET3-PLC

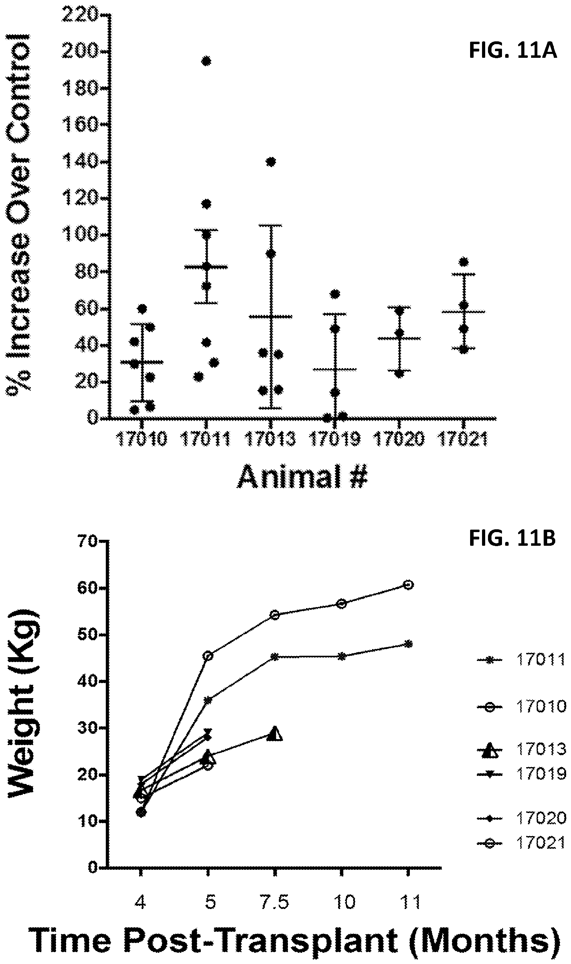

To test the therapeutic potential of an in utero treatment for HA, PLCs from MCB 103 were transduced with EF1α-mcoET3 LV, expanded, and qPCR was performed with vector-specific primers, comparing amplification to that from a known single-copy gene. These analyses yielded a vector copy number of 0.35±0.05 per diploid genome equivalent. These cells were prenatally transplanted (PNTx) into sheep fetuses (n=12) at a dose of 10⁷-10⁸ cells/kg fetal weight, at 65 gestation days (gd; term 145 gd), the equivalent of 16-17 weeks gestation in humans, using a clinically-employed ultrasound-guided transcutaneous injection procedure. Following birth, plasma FVIII activity levels were measured by aPTT in 6 animals, at intervals from 1-14 months post-PNTx, and compared to levels present in control, non-transplanted animals. Animals treated prenatally with mcoET3-transduced PLCs had significantly increased activity levels of plasma FVIII when compared to control, non-transplanted animals. Data from this analysis are shown in **FIG. 11A****.** Elevated plasma FVIII levels were maintained for at least 1-year post-birth, despite the rapid growth curve in these animals. 1-year post-birth, sheep weigh roughly 120 lbs, which is the equivalent of a young adult human **(****FIG. 11B****).** ELISAs using an antibody specific to ET3-FVIII were used to confirm the results obtained by aPTT (data not shown).

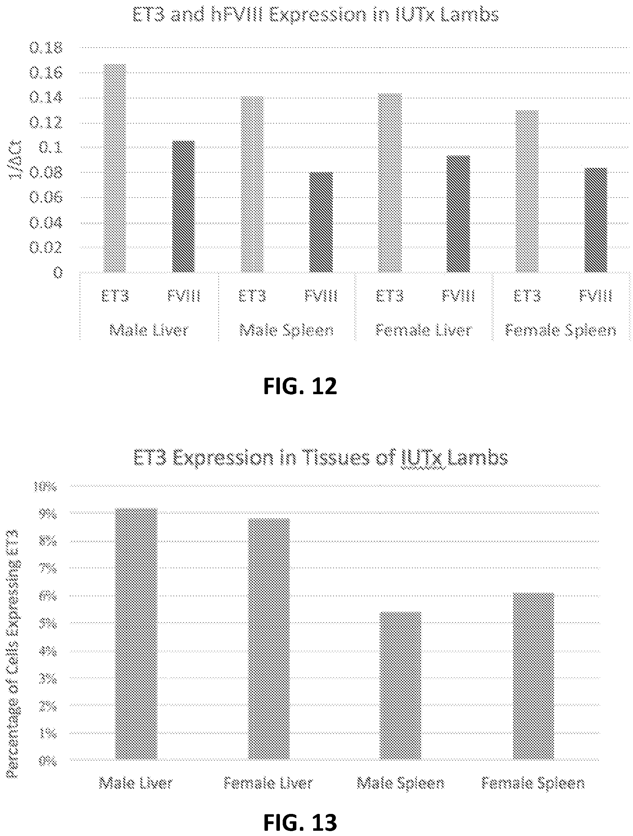

The locations of engraftment of the PLC-mcoET3 after PNTx were identified by detecting the presence of mRNA transcripts of both mcoET3 and human FVIII in tissues from lambs that were treated prenatally with PLC-mcoET3. Lambs that received PNTx were euthanized at birth, and tissues were removed. RNA was extracted from the tissues using a commercially available kit (Qiagen) and reverse-transcribed to cDNA. The cDNA was then analyzed by qPCR with primers specific to the mcoET3 transgene and to the endogenous housekeeping gene GAPDH. The DeltaDelta Ct method was then used to establish the amount of mcoET3 RNA present in each tissue relative to the levels of GAPDH. These analyses demonstrated that, in both the liver and spleen, levels of mcoET3 FVIII mRNA transcripts were substantially higher than those of the endogenous human FVIII, demonstrating that the transgene was not silenced and effectively produced mcoET3 **(****FIG. 12****).**

The number of PLCs that had engrafted within the liver and spleen were determined. Briefly, a standard curve was generated by mixing known number of mcoET3-transduced PLCs with normal untransduced human NK cells (which do not express FVIII) at varying ratios, isolating RNA, and performing qPCR with primers specific to the mcoET3 transgene. RNA isolated form the liver and spleen of the PNTx animals were run simultaneously. The levels of mcoET3 RNA within these tissues were then quantified using the resultant Ct values to extrapolate from the standard curve and thereby, determine the number of mco-ET3-expressing cells within each tissue **(****FIG. 13****).** Roughly 9% of cells within the liver at 5-6% of cells within the spleen of the PNTx-treated animals expressed mcoET3. Considering that the total cell mass of the liver at this point in development is roughly 30 billion cells, 9% represents a large number of mcoET3-expressing cells.

### Example 9. In Utero Transplantation

Having determined the cellular delivery strategies that yield the highest engraftment and the most efficient production/secretion of lentivector-encoded FVIII following IUTx in healthy fetuses, we will test the ability of IUTx to achieve long-term correction in HA sheep, using clinical parameters and coagulation assays to assess whether IUTx is curative or converts severe HA to a mild or moderate phenotype. We will perform IUTx with AFPs modified with the EF1α-[oFVIII]-IRES-GFP SIN lentivector comprising the polynucleotide sequence set forth in SEQ ID NO: 33 **(****FIG. 2****)** or MSPs modified with the EF1α-[ET3]-IRES-GFP SIN lentivector comprising an ET3 sequence having the polynucleotide sequence set forth in SEQ ID NO: 11. **(****FIG. 3****).**

To accomplish this goal, 20 HA carriers will be bred or artificially inseminated via laparoscopy, as we have been doing, with the support of the North Carolina State Theriogenology team. At 35-45 days of gestation (term: 145 days), the resultant fetuses will be screened in utero for the HA mutation using a PCR-based RFLP on fetal cells within the amniotic fluid as described in Porada, C.D., et al., Clinical and molecular characterization of a re-established line of sheep exhibiting hemophilia A. J Thromb Haemost, 2010. 8(2):276-85. *Autologous* AFPs will be grown from each fetus that the RFLP reveals to carry the HA mutation, and maternal (*haploidentical*) or *allogeneic* MSPs will be isolated and cultured as described in Example 2. Cells will be grown to 70% confluence and transduced with the AFPs modified with the EF1α-[oFVIII]-IRES-GFP SIN lentivector **(****FIG. 2****)** or MSPs modified with the EF1α-[ET3]-IRES-GFP SIN lentivector **(****FIG. 3****)** as discussed in Examples 2 and 3, above.

After transduction, an aliquot of cells will be frozen and stored for vector copy number and transduction efficiency assessment by qPCR, and for subsequent infusions to be given postnatally (if needed to boost FVIII levels). These cells will then be transplanted into PCR-confirmed HA fetuses (n=5-6 per cell type) at 60-65 days of gestation, using a dose of 10⁸ cells/kg of the subject's weight in a 0.5 ml volume. The transplantation will be made into the fetal peritoneum by percutaneous injection under continuous real-time ultrasound visualization. The fetal heart rate pre- and post-transplant will be compared to ensure fetal well-being. Since the HA sheep phenotypically equal or exceed the bleeding phenotype seen in humans with severe HA, the use of this model will allow us to accurately define the risk of bleeding after IUTx for HA. To address this clinically important issue, we will examine the fetal abdomen by ultrasound immediately after transplant, and at 72-96 hours post-IUTx, for evidence of bleeding, which would manifest within this timeframe as complex peritoneal ascites.

Five to six fetuses are being used per experimental group to ensure differences observed in clinical parameters and/or circulating FVIII levels are statistically significant and reproducible, using the statistical calculations detailed in prior Examples.

Following IUTx, ewes will be allowed to carry the pregnancy to term. When the sheep have nearly completed gestation, the pregnant ewes will be placed under close observation, and ewes will either be induced into labor for natural delivery, or the lambs will be delivered by C-section. We have used both approaches with success.

At birth, we will first determine the therapeutic impact of this approach by observing the bleeding symptoms, which should improve with even low-level FVIII production by the transplanted MSPs or AFPs. In contrast to other HA animal models, the phenotype of these HA animals is remarkably severe, with fully affected, untreated sheep dying within hours/days of birth, from prolonged umbilical cord and intra-abdominal bleeding. Any therapeutic benefit resulting from IUTx will thus be readily apparent within moments of birth.

Shortly after birth, platelet-deficient plasma will be collected and WBCT, aPTT, and PT performed by the clinical hematology laboratory at Wake Forest Baptist Medical Center. We will also quantify plasma FVIII levels in the IUTx sheep using a sensitive chromogenic assay (diaPharma), and the levels of lentivector-encoded FVIII protein by ELISA, as detailed in Examples 2 and 3, comparing values from the IUTx treated HA lambs to those of untreated HA sheep, using as a reference/control, a panel of non-carrier females and unaffected males. Aliquots of each plasma sample will be frozen and independent confirmation/validation of all coagulation tests, ELISA, and chromogenic assays will be performed.

We will continue to monitor the sheep for bleeds, and the coagulation tests, ELISA, and chromogenic assay will be repeated monthly until at least one year of age. This repeated sampling will allow us to answer two key questions: 1) what levels of vector-encoded FVIII are expressed as a result of IUTx; and 2) how long do these levels of FVIII persist. We will also use RFLP to confirm that all clinically normal newborns are in fact genetically hemophiliac. We will also confirm that the patterns and levels of cell engraftment obtained in HA animals are similar to those obtained in normal animals, by evaluating tissues from IUTx-treated HA animals (at euthanasia) using the methods detailed in Examples 2 and 3.

### Example 10. Assess Long-Term Tolerance to Factor VIII

To address whether IUTx induces durable immune tolerance to the lentivector-encoded FVIII, allowing postnatal factor infusion without inhibitor induction, we will first perform studies on all of the IUTx-treated HA lambs in Example 4 (n=10-12) after birth. Beginning at birth, and monthly thereafter, all sheep will have blood samples drawn for standard coagulation tests, chromogenic assays to quantify FVIII levels/activity, and ELISA to test for antibodies to FVIII, as described above. If IUTx has induced tolerance, these tests should reveal stable circulating FVIII levels and absence of FVIII antibodies.

Studying the factors triggering the onset of inhibitors in HA, and developing methods to avoid their formation or eliminate them once they have formed, has been difficult in animal models. In human patients at risk of developing high-titer inhibitors to FVIII, the onset of inhibitors is preceded by a shift in the relative distribution of IgG subclasses, with the appearance of FVIII-specific IgG4 antibodies. Importantly, these IgG4 antibodies are only present in patients who develop inhibitors; they are absent in healthy individuals and in HA patients who lack inhibitors. While mice have been the mainstay of inhibitor research, they do not have/produce the IgG4 subclass. Canine models do not produce inhibitors to canine Factor VIII. Sheep, like humans, produce the IgG4 subclass, and they perform the same immunological functions in sheep as in man. HA sheep are thus a unique model in which to study this clinically important aspect of inhibitor formation. Since even normal/healthy people within the general population can harbor low levels of circulating antibodies to FVIII of the IgG1 subclass, we will also use an IgG4-specific ELISA (MyBioSource) to screen all of the IUTx recipients for the presence/emergence of IgG4 immunoglobulins with samples from each of the monthly blood draws. If we detect significant IgG4 levels in any lambs (only present at very low levels without a specific trigger [197]), we will purify the IgG4 using the CaptureSelect^{™} IgG4 Affinity Matrix (ThermoFisher Scientific). We will then test the purified IgG4 for its ability to bind recombinant oFVIII or ET3, depending upon which transduced cells the animal received via IUTx.

To determine if the IUTx-induced state of hypo-responsiveness/tolerance allows postnatal FVIII infusion without antibodies/inhibitors, we will do the following study. At ~6 months of age, once we have established baseline plasma FVIII levels and the levels of antibodies to FVIII (very low/absent if tolerized), we will select animals (n=5-6) with the lowest circulating levels of FVIII, and administer recombinant oFVIII or ET3 (depending upon which transduced cells the animal received via IUTx). We will dose the sheep intravenously to achieve a circulating FVIII activity level of 80-100%, as we typically do when treating these sheep for severe bleeds. We will administer a total of 7 doses of recombinant oFVIII or ET3, spaced 4 days apart, as this is the frequency with which HA sheep normally require treatment for spontaneous bleeds. Prior to, and at 10 and 30 minutes; 1, 3, 6, 9, 24, 48, 72, and 96 hours post-infusion, plasma FVIII activity will be measured by chromogenic assay (Diapharma). The FVIII activity observed in each individual sheep at each time point will then be fed into WinNonLin software (Pharsight, Cary, NC) to calculate standard pharmacokinetic parameters, including maximum activity (Cmax), t1/2, clearance (CL), volume of distribution at steady-state (Vss), and mean residence time (MRT). If FVIII activity drops with subsequent injections or clearance is accelerated, we will surmise that antibodies have formed in these sheep. ELISA will also establish the kinetics of antibody formation. At one week after the final injection, we will again collect blood to assess the FVIII plasma levels/activity and to obtain serum. If ELISA reveals the presence of antibodies to FVIII, we will ascertain whether these antibodies are inhibitory, and their levels, using the Nijmegen modified Bethesda assay and a commercial kit (Technoclone/ DiaPharma Group, Inc.), and will use the same methods described in detail above to ascertain whether an IgG4 response is present. We will also use an IgM-specific ELISA to determine whether the IgG response is preceded by a primary IgM response, as occurs in human HA patients who form FVIII inhibitors.

Successful infusion of FVIII in therapeutic doses without inhibitors could mean that IUTx induced immune tolerance, or that immune hyporesponsiveness has occurred. The latter, under the right conditions (such as infection, trauma, inflammation) could trigger immunity to FVIII. To distinguish between these possibilities, we will attempt to break IUTx-induced tolerance by challenging 3 IUTx recipients (that received ET3-transduced MSPs as described in Example 3) with ET3 (SEQ ID NO: 11). We will administer subcutaneous injections, at 6 separate sites, of 8 mg recombinant ET3 (rET3) in Freunds complete adjuvant. This immunization procedure will be repeated 3 weeks later, with the same dose of ET3, but in Freunds incomplete adjuvant. As a negative control, we will include 3 naive HA sheep immunized with ovalbumin, and as a positive control, we will include 3 non-IUTx-treated HA sheep challenged with rET3. Ten days following the second injection, we will collect blood to assess FVIII plasma levels/activity and to ascertain whether antibodies have formed in these sheep following immunization with rET3 and, if so, whether these antibodies are inhibitory, as detailed in above. As before, we will perform studies to examine the appearance of IgM, and to assess whether ET3-specific IgG4 antibodies have developed following this unforgiving immunization protocol.

While the preceding studies on IgG formation will provide valuable information, FVIII-specific memory B cells can now be isolated and studied *in vitro* as described in Allacher et al., Stimulation and inhibition of FVIII-specific memory B-cell responses by CpG-B (ODN 1826), a ligand for Toll-like receptor 9. Blood, 2011. 117(1):259-67; Hausl et al., High-dose factor VIII inhibits factor VIII-specific memory B cells in hemophilia A with factor VIII inhibitors. Blood, 2005. 106(10):3415-22; and Hausl et al., Preventing restimulation of memory B cells in hemophilia A: a potential new strategy for the treatment of antibody-dependent immune disorders. Blood, 2004. 104(1):115-22. We will use these previously published methods to assess the presence of memory B cells in these animals, and test their ability to respond to FVIII *in vitro* and differentiate into antibody-secreting cells, using an established ELISpot assay system to quantify the numbers of FVIII-specific B cells present in these animals following immunization with ET3 in adjuvant. As these prior (murine) studies used splenocytes, which requires euthanizing the animals, we will substitute marrow cells for splenocytes, as this works equally well in these assays and marrow aspirates can easily be collected from the sheep. To assess the presence of a T cell response to ET3 in these animals following challenge, CD4+CD25- cells (T-helpers) and CD4+CD25+ cells (Tregs) will be isolated from peripheral blood by immunomagnetic bead sorting (Miltenyi) and cultured for 7 days in the presence of recombinant ET3 or concanavalin A (positive control), and their proliferation quantitated using a colorimetric BrdU assay as described in Boura, J.S., et al., Evaluation of gene delivery strategies to efficiently overexpress functional HLA-G on human bone marrow stromal cells. Mol Ther Methods Clin Dev, 2014. 2014(1):14041. Transcript levels of cytokines in an aliquot of these cells will also be measured by qRT-PCR using a commercial array (SA Bioscience), paying particular attention to IL-4, IL-6, IL-10, TGF-β, and IFN-γ. ELISpot assays will also be performed to detect hFVIII-specific IL-4 and IFN-γ responses, using Bovine ELISpot Development Modules (R&D Systems). SEB and PMA/Ionomycin will be used as positive controls.

### Example 11. In Utero Transplantation in Sheep with Placental MSC

We propose to use human placental stem cells (PLSC) (MSC derived from placental tissue) that have been engineered to express a B-domain-deleted, expression-optimized coagulation factor VIII (FVIII) transgene (tPLSC) for treating hemophilia A (the ET3 Factor VIII modified transgene, or one of its codon-optimized variants). These cells will be tested in a preclinical large animal (sheep) for both their safety and their therapeutic efficacy in correcting hemophilia A prior to birth.

Placenta Tissue Procurement: The critical raw material is a full-term placenta which was obtained during delivery after informed consent from the mother. Immediately following delivery, the tissue specimen was placed in a labeled, sterile container at ambient temperature. The container was sealed, labeled, placed in an insulated cooler with 15-25°C phase change material to maintain temperature and transported to the manufacturing facility by courier. A medical and behavioral health history assessment along with qualified serologic results from the mother were used to accept the tissue for use.

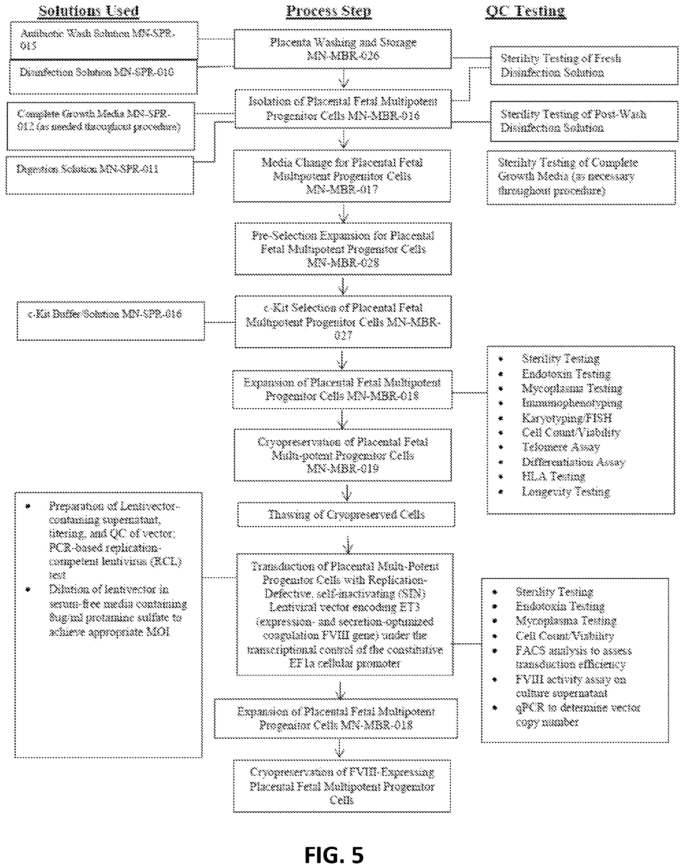

Isolation of MSC from Placenta: Placenta samples collected were transported to our GMP-compliant facility. Isolated placenta-derived cells were subsequently plated in Alpha-MEM supplemented with 17% AmnioMax Basal media, 15% FBS, 2.5 µg/mL gentamicin, 2% AmnioMax supplement, and 1% Glutamax to obtain P-MSCs, in a protocol modified from the literature. (See Semenov, O.V., et al., Multipotent mesenchymal stem cells from human placenta: critical parameters for isolation and maintenance of sternness after isolation. Am J Obstet Gynecol. 2010, 202(2):193.e1-193.e13, and Nazarov I.L., et al., Multipotent stromal stem cells from human placenta demonstrate high therapeutic potential, Stem Cells Transl Med. 2012; 1(5):359-72.) The cells were allowed to proliferate *in vitro* and were maintained in culture for about 2-3 weeks. Cells were passaged with TrypLE each time the cultures reach 70% confluence, until sufficient cells were obtained. Cells from placenta were then analyzed, using FACS, for expression of c-kit (also referred to as CD117), CD34, CD90, and CD133. C-kit positive cells, were immunomagnetically selected for c-kit. Selection was performed on at least 1 × 10⁶ cells using Miltenyi C-Kit Selection microbeads following the manufacturer's protocol. After c-kit selection, the cells were plated in tissue culture treated flasks and grown to 70% confluency. Cells were grown and expanded as described above until 5×10⁸ cells were obtained (typically 5 to 7 total passages). To cryopreserve, cells were harvested using the appropriate cell expansion protocol and resuspended in a 4°C solution of CryoStor 10 (Biolife Solutions) freezing medium at a concentration of approximately 1 ×10⁷ cells/ml. Aliquots were transferred to cryovials, frozen using a controlled rate freezer, and stored in the vapor phase of a liquid nitrogen freezer. To thaw, cryovials were rapidly defrosted in a 37°C water bath, or thawed using an automated thawing system such as the Thawstar (BioCision / MedCision), and the contents diluted 1:10 in the appropriate growth media at 4°C. Cells were centrifuged and resuspended in fresh 37°C growth media. Thawed cells were cultured for one passage prior to being used. Expanded cells were thoroughly characterized at low and high doses in nude rats to assess the potential tumorigenicity and toxicology at 16 weeks (112 days) and cryopreserved as a Master Cell Bank (MCB) available for future use. See Flowchart in **FIG. 5****.** Cell line PLS-15-103 was generated as a MCB line using this process.

Transduction of Human PLCs: Once thawed and the c-kit positive cells reach 70% confluency, cells will be washed and transduced under serum-free conditions with a GMP-grade HIV-based, replication-defective lentivector (MOI=10-50; in the presence of 4-8µg/ml protamine sulfate. The lentiviral vector will be manufactured by the Cincinnati Children's Hospital GMP Vector Core (https://research.cchmc.org/translationalcores/vector-production/gmp-vector-production).

The lentiviral vector will encode a B domain-deleted (BDD) expression-optimized FVIII transgene **(****FIG. 4****),** which is the ET3 transgene (or one of its codon-optimized variants) having the polynucleotide sequence set forth in SEQ ID NO: 11 and vector described in this disclosure above in prior Examples. This vector contains the constitutively active human EF1α promoter driving expression of FVIII. The vector construct also contains the 3'-modified long terminal repeat (LTR) resulting in a self-inactivating (SIN) lentivector. Using a SIN vector and the EF1α promoter, which cannot transactivate neighboring cellular genes, adds 2 safeguards against the theoretical risk of insertional mutagenesis. After 48 hours of transduction, the cell suspension will be washed and cells will be expanded for extensive characterization and re-banking. At the time of treatment, cells will be thawed, cultured for one passage to allow for recovery, harvested and re-suspended in serum-free media (QBSF-60, Quality Biologicals) for in utero injection, as detailed below.

In Vivo Pharmacology: The FVIII-expressing human PLSC (tPLSC) will be transplanted into the fetal peritoneal cavity and the fetus will be monitored until it reaches full-term for delivery.

In Utero Transplantation in HA Sheep: At 60-75 days of gestation (term: 145 days), general anesthesia will be induced with Telazol (tiletamine hydrochloride 50 mg/mL, zolazepam hydrochloride 50 mg/mL) or dexmedetomidine/ketamine, intravenously or intramuscularly, and, after intubation (or masking), the ewes will be maintained on 3% sevoflurane in oxygen. Once an adequate state of sedation/anesthesia is achieved, FVIII-expressing human tPLSC will then be transplanted into the peritoneal cavity of the fetus (n=2-3 fetuses) by percutaneous, ultrasound-guided injection using a 6", 22-gauge EchoBlock^{®} laser-etched ultrasound needle (Havel's^{®} by Hakko^{®} Medical, Japan). Based on our previous preclinical studies in sheep, we will use a dose of 1-10×10⁷ tPLSC /kg fetal weight, and will employ an injection volume of <1 mL. To ensure correct needle placement within the peritoneal cavity, the needle will be inserted through the anterior abdominal wall of the fetal sheep superior and lateral to the fetal bladder to avoid the umbilical arteries, and we will watch for microbubbles moving within the peritoneal cavity as the cells are instilled. Following cell injection, fetal well-being will be confirmed by ultrasound examination, and the fetuses will then be allowed to complete term normally.

Post-Delivery Assessment: Treated lambs will be delivered at full-term under close veterinary observation. At birth, the first thing we will examine to assess the therapeutic potential of this approach is the bleeding symptoms, which should improve as a result of FVIII production by the engrafted tPLSC. Given the severe phenotype of these animals (fully affected sheep die within hours/days of birth), any benefit resulting from IUTx will be readily apparent. Shortly after birth, platelet-deficient plasma will be collected and WBCT, aPTT, and PT performed as described above in prior Examples.

We will also quantify plasma FVIII levels in the IUTx sheep using a highly sensitive chromogenic assay (diaPharma). Values from the IUTx-treated HA lambs will be compared to previously published values from untreated HA sheep and to a reference panel of non-carrier females and unaffected males. The sheep will be continually monitored for bleeds, and the coagulation tests and chromogenic assay will be repeated each month throughout the course of this proof-of-principle preclinical study. This repeated sampling will allow us to answer two key questions: 1) what levels of vector-encoded FVIII are expressed as a result of IUTx; and 2) how long these levels of FVIII persist. Given that IUTx should presumably produce some clinical benefit, we will use the PCR-based RFLP as described in prior Examples to confirm that clinically normal newborns are indeed genetically hemophiliac.

### Example 12. In Utero Transplantation in Humans with Placental MSC

The proposed study will involve 5 expectant families with a history of hemophilia A who have elected to undergo prenatal genetic screening to determine whether their unborn child is affected with hemophilia A, and who consent to participating in a trial to test the intrauterine transplantation therapy (IUTx).

*Allogeneic* cells from a previously established Master Cell Bank that have been engineered to constitutively express FVIII as described in Example 4 will be used. Beyond existing data on these cells, extensive characterization on transduced cells (tPLC) will be done prior to re-banking, including integration copy number, genomic stability and verification of FVIII secretion into the culture media of the cells (by Activated Partial Thromboplastin Time [aPTT] assay). One week before the day of infusion, the gene-engineered cells will be thawed and expanded. On the day of infusion, cultures will be harvested, washed once, and resuspended in <1mL of serum-free media (QBSF-60, Quality Biologicals, Inc.). They will then be infused into the peritoneal cavity of the fetus by percutaneous, ultrasound-guided injection using a 6", 22-gauge EchoBlock^{®} laser-etched ultrasound needle (Havel's^{®} by Hakko^{®} Medical, Japan). Based on our preclinical studies in sheep, we will use a dose of 1-10×10⁷ tPLC/kg fetal weight. Patients will remain overnight in the hospital for monitoring post-procedure. They will continue with the standard of care prenatal visit schedule for pregnancies complicated by hemophilia under the care of a maternal-fetal medicine specialist. At term, the child will be delivered by Caesarian section to prevent any potential trauma to the affected child as a result of pressure/squeezing during vaginal delivery. Immediately following delivery, a small volume of umbilical cord blood will be collected and analyzed by Activated Partial Thromboplastin Time (aPTT) Test or Chromogenix assay to precisely quantitate the levels of FVIII in the circulation and thereby ascertain the efficacy of the in utero treatment. FVIII levels will be assessed each 2-3 weeks during the first 4 months of life and monthly thereafter to determine the duration of the correction afforded by this in utero treatment. Based upon extensive preclinical animal work, we anticipate that correction should be lifelong. Our goal is to achieve circulating FVIII levels ≥5% of normal, as this would ensure the child presents with a mild, essentially normal phenotype.

All features of the described systems are applicable to the described methods *mutatis mutandis*, and vice versa.

It is to be understood that the figures and descriptions of the disclosure have been simplified to illustrate elements that are relevant for a clear understanding of the disclosure. It should be appreciated that the figures are presented for illustrative purposes and not as construction drawings. Omitted details and modifications or alternative embodiments are within the purview of persons of ordinary skill in the art.

It can be appreciated that, in certain aspects of the disclosure, a single component may be replaced by multiple components, and multiple components may be replaced by a single component, to provide an element or structure or to perform a given function or functions. Except where such substitution would not be operative to practice certain embodiments, such substitution is considered within the scope of the disclosure.

## Claims

1. Modified mesenchymal stem/stromal cells (MSC) for use in a method of treating a subject prenatally diagnosed as having hemophilia, the method comprising injecting said modified MSC into the subject *in utero,* wherein the modified MSC are modified, via introduction of a transgene or genome editing, to express high levels of Factor VIII protein or high levels of both Factor VIII protein and von Willebrand factor (vWF) protein, wherein the Factor VIII gene sequence comprises one or more genetic modifications that increase protein expression, protein stability, or both, and wherein the modified MSC are c-kit expressing modified placental tissue MSC.

2. The MSC for the use of any one of the preceding claims, wherein the modified MSC express c-kit, CD34, CD90, and CD133; and/or wherein the modified MSC comprise modified autologous placental MSC.

3. The MSC for use of any one of the preceding claims,
wherein the Factor VIII gene sequence comprises human Factor VIII gene sequence, ovine Factor VIII, or porcine Factor VIII gene sequence;
wherein the Factor VIII gene sequence comprises a B domain;
wherein the Factor VIII gene sequence comprises modifications to reduce immunogenicity of the expressed Factor VIII protein;
wherein the Factor VIII gene sequence is a human Factor VIII gene sequence, and wherein one or more human Factor VIII protein domain sequences are substituted with the sequence of a corresponding one or more porcine Factor VIII protein domain sequences; and/or
wherein the Factor VIII gene sequence comprises human Factor VIII A2 and C2 domains and porcine Factor VIII Al, A3, and Cl domains.

4. The MSC for the use of any one of the preceding claims, wherein the vWF gene sequence comprises at least one of human vWF gene sequence, ovine vWF, or porcine vWF gene sequence; and/or wherein the vWF sequence comprises one or more genetic modifications that increase protein expression, protein stability, or both.

5. The MSC for the use of any one of the preceding claims, wherein the modified MSC comprise a viral vector, the viral vector comprising a Factor VIII gene sequence operatively linked to a constitutively active promoter.

6. The MSC for the use of any one of claims 1-4, wherein the modified MSC comprise one or more modifications to an endogenous Factor VIII gene sequence that increases protein expression, protein stability, or both, of the Factor VIII protein.

7. The MSC for the use of any one of the preceding claims, wherein the modified MSC comprise a viral vector comprising a vWF gene sequence operatively linked to a constitutively active promoter.

8. The MSC for the use of any one of any one of claims 1-5 and 8, wherein the modified MSC a comprise a viral vector comprising a Factor VIII gene sequence operatively linked to a constitutively active promoter and a vWF gene sequence operatively linked to a constitutively active promoter, optionally wherein the Factor VIII gene sequence and the vWF gene sequence are operatively linked to the same constitutively active promoter.

9. The MSC for the use of any one of claims 1-6, wherein the modified MSC comprise one or more modifications to an endogenous vWF gene sequence that increases protein expression, protein stability, or both, of the vWF protein.

10. The MSC for the use of any one of the preceding claims, wherein the modified MSC are generated by modifying isolated MSC to express high levels of Factor VIII protein or high levels of both Factor VIII protein and von Willebrand factor (vWF) protein, and wherein the isolated MSC are isolated from placental tissue, and optionally wherein the isolated MSC are isolated prenatally from samples obtained from the subject's mother; and optionally
wherein the modified MSC are generated by introducing a viral vector into the isolated MSC, the viral vector comprising a Factor VIII gene sequence operatively linked to a constitutively active promoter.

11. The MSC for the use of claim 10, wherein the modified MSC are generated by modifying the isolated MSC via gene editing of an endogenous Factor VIII gene sequence to introduce one or more modifications to the endogenous Factor VIII gene sequence that increase protein expression, protein stability, or both, of the Factor VIII protein.

12. The MSC for the use of any one of claims 10-11, wherein the modified MSC are generated by introducing a viral vector into the isolated MSC, the viral vector comprising a vWF gene sequence operatively linked to a constitutively active promoter.

13. The MSC for the use of any one of claims 10 and 12, wherein the modified MSC are generated by introducing a viral vector into the isolated MSC, the viral vector comprising a Factor VIII gene sequence operatively linked to a constitutively active promoter and a vWF gene sequence operatively linked to a constitutively active promoter; and optionally wherein the Factor VIII gene sequence and the vWF gene sequence are operatively linked to the same constitutively active promoter.

14. The MSC for the use of any one of claims 10-11, wherein the modified MSC are generated by modifying the isolated MSC via gene editing of an endogenous vWF gene sequence to introduce one or more modifications to the endogenous vWF gene sequence that increase protein expression, protein stability, or both, of the vWF protein.

15. The MSC for the use of any of the preceding claims, wherein the method comprises injecting the modified MSC into the subject *in utero* via intraperitoneal injection; and/or wherein the modified MSC are injected into the subject at least once, at least twice, or at least three times; and/or wherein the modified MSC are injected into the subject in an amount of about 10⁷-10⁹ MSC per kilogram weight of the subject.

## Patentansprüche

1. Modifizierte mesenchymale Stamm-/Stromazellen (MSC) zur Verwendung in einem Verfahren zum Behandeln eines Subjekts, bei dem pränatal Hämophilie diagnostiziert wurde, wobei das Verfahren die Injektion der modifizierten MSC *im Mutterleib* in den Patienten umfasst, wobei die modifizierten MSC durch Einführung eines Transgens oder Genomeditierung modifiziert werden, um hohe Konzentrationen des Faktor-VIII-Proteins oder hohe Konzentrationen sowohl des Faktor-VIII-Proteins als auch des von-Willebrand-Faktor-Proteins (vWF) exprimieren, wobei die Faktor-VIII-Gensequenz eine oder mehrere genetische Modifikationen umfasst, die die Proteinexpression, die Proteinstabilität oder beides erhöhen und wobei die modifizierten MSC c-kit exprimierende modifizierte MSC aus Plazentagewebe sind.

2. MSC zur Verwendung nach einem der vorstehenden Ansprüche, wobei die modifizierten MSC c-kit, CD34, CD90 und CD133 exprimieren; und/oder wobei die modifizierten MSC modifizierte autologe Plazenta-MSC umfassen.

3. MSC zur Verwendung nach einem der vorstehenden Ansprüche,
wobei die Faktor-VIII-Gensequenz eine humane Faktor-VIII-Gensequenz, eine ovine Faktor-VIII- oder eine porcine Faktor-VIII-Gensequenz umfasst;
wobei die Faktor-VIII-Gensequenz eine B-Domäne umfasst;
wobei die Faktor-VIII-Gensequenz Modifikationen zur Verringerung der Immunogenität des exprimierten Faktor-VIII-Proteins umfasst;
wobei die Faktor-VIII-Gensequenz eine humane Faktor-VIII-Gensequenz ist und wobei eine oder mehrere humane Faktor-VIII-Proteindomänensequenzen durch die Sequenz einer oder mehrerer entsprechender porciner Faktor-VIII-Proteindomänensequenzen ersetzt sind; und/oder
wobei die Faktor-VIII-Gensequenz humane Faktor-VIIIA2- und -C2-Domänen und porcine Faktor-VIII-AI-, -A3- und -C1-Domänen umfasst.

4. MSC zur Verwendung nach einem der vorstehenden Ansprüche, wobei die vWF-Gensequenz mindestens eine umfasst von: humaner vWF-Gensequenz, oviner vWF-Gensequenz oder porciner vWF-Gensequenz; und/oder wobei die vWF-Sequenz eine oder mehrere genetische Modifikationen umfasst, die die Proteinexpression, die Proteinstabilität oder beides erhöhen.

5. MSC zur Verwendung nach einem der vorstehenden Ansprüche, wobei die modifizierten MSC einen viralen Vektor umfassen, wobei der virale Vektor eine Faktor-VIII-Gensequenz umfasst, die operativ mit einem konstitutiv aktiven Promotor verknüpft ist.

6. MSC zur Verwendung nach einem der Ansprüche 1 bis 4, wobei die modifizierten MSC eine oder mehrere Modifikationen einer endogenen Faktor-VIII-Gensequenz umfassen, die die Proteinexpression, Proteinstabilität oder beides des Faktor-VIII-Proteins erhöhen.

7. MSC zur Verwendung nach einem der vorstehenden Ansprüche, wobei die modifizierten MSC einen viralen Vektor umfassen, der eine vWF-Gensequenz umfasst, die operativ mit einem konstitutiv aktiven Promotor verknüpft ist.

8. MSC zur Verwendung nach einem der Ansprüche 1 bis 5 und 8, wobei die modifizierten MSC einen viralen Vektor umfassen, der eine Faktor-VIII-Gensequenz, die operativ mit einem konstitutiv aktiven Promotor verknüpft ist, und eine vWF-Gensequenz, die operativ mit einem konstitutiv aktiven Promotor verknüpft ist, umfasst, wobei optional die Faktor-VIII-Gensequenz und die vWF-Gensequenz operativ mit demselben konstitutiv aktiven Promotor verknüpft sind.

9. MSC zur Verwendung nach einem der Ansprüche 1 bis 6, wobei die modifizierten MSC eine oder mehrere Modifikationen einer endogenen vWF-Gensequenz umfassen, die die Proteinexpression, Proteinstabilität oder beides des vWF-Proteins erhöhen.

10. MSC zur Verwendung nach einem der vorstehenden Ansprüche, wobei die modifizierten MSC durch Modifizierung isolierter MSC erzeugt werden, um hohe Konzentrationen an Faktor-VIII-Protein oder hohe Konzentrationen sowohl an Faktor-VIII-Protein als auch an von-Willebrand-Faktor-Protein (vWF) zu exprimieren, und wobei die isolierten MSC aus Plazentagewebe isoliert werden und wobei optional die isolierten MSC pränatal aus Proben isoliert werden, die von der Mutter des Subjekts gewonnen wurden; und optional
wobei die modifizierten MSC durch Einführen eines viralen Vektors in die isolierten MSC erzeugt werden, wobei der virale Vektor eine Faktor-VIII-Gensequenz umfasst, die operativ mit einem konstitutiv aktiven Promotor verknüpft ist.

11. MSC zur Verwendung nach Anspruch 10, wobei die modifizierten MSC durch Modifizierung der isolierten MSC mittels Geneditierung einer endogenen Faktor-VIII-Gensequenz erzeugt werden, um eine oder mehrere Modifikationen an der endogenen Faktor-VIII-Gensequenz einzuführen, die die Proteinexpression, Proteinstabilität oder beides des Faktor-VIII-Proteins erhöhen.

12. MSC zur Verwendung nach einem der Ansprüche 10 bis 11, wobei die modifizierten MSC durch Einführen eines viralen Vektors in die isolierten MSC erzeugt werden, wobei der virale Vektor eine vWF-Gensequenz umfasst, die operativ mit einem konstitutiv aktiven Promotor verknüpft ist.

13. MSC zur Verwendung nach einem der Ansprüche 10 und 12, wobei die modifizierten MSC durch Einführen eines viralen Vektors in die isolierten MSC erzeugt werden, wobei der virale Vektor eine Faktor-VIII-Gensequenz, die operativ mit einem konstitutiv aktiven Promotor verknüpft ist, und eine vWF-Gensequenz, die operativ mit einem konstitutiv aktiven Promotor verknüpft ist, umfasst; und wobei optional die Faktor-VIII-Gensequenz und die vWF-Gensequenz operativ mit demselben konstitutiv aktiven Promotor verknüpft sind.

14. MSC zur Verwendung nach einem der Ansprüche 10 bis 11, wobei die modifizierten MSC durch Modifizierung der isolierten MSC mittels Geneditierung einer endogenen vWF-Gensequenz erzeugt werden, um eine oder mehrere Modifikationen an der endogenen vWF-Gensequenz einzuführen, die die Proteinexpression, Proteinstabilität oder beides des vWF-Proteins erhöhen.

15. MSC zur Verwendung nach einem der vorstehenden Ansprüche, wobei das Verfahren das Injizieren der modifizierten MSC in das Subjekt *im Mutterleib* durch intraperitoneale Injektion umfasst; und/oder wobei die modifizierten MSC dem Subjekt mindestens einmal, mindestens zweimal oder mindestens dreimal injiziert werden; und/oder wobei die modifizierten MSC dem Subjekt in einer Menge von etwa 10⁷-10⁹ MSC pro Kilogramm Gewicht des Subjekts injiziert werden.

## Revendications

1. Cellules souches/stromales mésenchymateuses (CSM) modifiées destinées à être utilisées dans un procédé de traitement d'un sujet ayant reçu un diagnostic prénatal d'hémophilie, le procédé comprenant l'injection desdites CSM modifiées dans le sujet *in utero,* dans lesquelles les CSM modifiées sont modifiées, par l'introduction d'un transgène ou l'édition génomique, pour exprimer des niveaux élevés de protéine de type facteur VIII ou des niveaux élevés à la fois de protéine de type facteur VIII et de protéine de type facteur von Willebrand (vWF), dans lesquelles la séquence du gène du facteur VIII comprend une ou plusieurs modifications génétiques qui augmentent l'expression de la protéine, la stabilité de la protéine, ou les deux, et dans lesquelles les CSM modifiées sont des CSM de tissu placentaire modifiées exprimant c-kit.

2. CSM destinées à être utilisées selon l'une quelconque des revendications précédentes, dans lesquelles les CSM modifiées expriment c-kit, CD34, CD90 et CD133 ; et/ou dans lesquelles les CSM modifiées comprennent des CSM placentaires autologues modifiées.

3. CSM destinées à être utilisées selon l'une quelconque des revendications précédentes,
dans lesquelles la séquence du gène du facteur VIII comprend la séquence du gène du facteur VIII humain, du facteur VIII ovin, ou la séquence du gène du facteur VIII porcin ;
dans lesquelles la séquence du gène du facteur VIII comprend un domaine B ;
dans lesquelles la séquence du gène du facteur VIII comprend des modifications pour réduire l'immunogénicité de la protéine de type facteur VIII exprimée ;
dans lesquelles la séquence du gène du facteur VIII est une séquence du gène du facteur VIII humain, et dans lesquelles une ou plusieurs séquences du domaine protéique du facteur VIII humain sont remplacées par la séquence parmi une ou plusieurs séquences du domaine protéique du facteur VIII porcin correspondantes ; et/ou
dans lesquelles la séquence du gène du facteur VIII comprend les domaines A2 et C2 du facteur VIII humain et les domaines Al, A3 et CI du facteur VIII porcin.

4. CSM destinées à être utilisées selon l'une quelconque des revendications précédentes, dans lesquelles la séquence du gène vWF comprend au moins l'une parmi la séquence du gène vWF humain, vWF ovin, ou la séquence du gène vWF porcin ; et/ou dans lesquelles la séquence du vWF comprend une ou plusieurs modifications génétiques qui augmentent l'expression de la protéine, la stabilité de la protéine, ou les deux.

5. CSM destinées à être utilisées selon l'une quelconque des revendications précédentes, dans lesquelles les CSM modifiées comprennent un vecteur viral, le vecteur viral comprenant une séquence du gène du facteur VIII liée de manière opérationnelle à un promoteur constitutivement actif.

6. CSM destinées à être utilisées selon l'une quelconque des revendications 1 à 4, dans lesquelles les CSM modifiées comprennent une ou plusieurs modifications d'une séquence endogène du gène du facteur VIII qui augmente l'expression de la protéine, la stabilité de la protéine, ou les deux, de la protéine de type facteur VIII.

7. CSM destinées à être utilisées selon l'une quelconque des revendications précédentes, dans lesquelles les CSM modifiées comprennent un vecteur viral comprenant une séquence du gène vWF liée de manière opérationnelle à un promoteur constitutivement actif.

8. CSM destinées à être utilisées selon l'une quelconque des revendications 1 à 5 et 8, dans lesquelles les CSM modifiées un comprennent un vecteur viral comprenant une séquence du gène du facteur VIII liée de manière opérationnelle à un promoteur constitutivement actif et une séquence du gène vWF liée de manière opérationnelle à un promoteur constitutivement actif, éventuellement dans lesquelles la séquence du gène du facteur VIII et la séquence du gène vWF sont liées de manière opérationnelle au même promoteur constitutivement actif.

9. CSM destinées à être utilisées selon l'une quelconque des revendications 1 à 6, dans lesquelles les CSM modifiées comprennent une ou plusieurs modifications d'une séquence endogène du gène vWF qui augmente l'expression de la protéine, la stabilité de la protéine, ou les deux, de la protéine de type vWF.

10. CSM destinées à être utilisées selon l'une quelconque des revendications précédentes, dans lesquelles les CSM modifiées sont générées en modifiant des CSM isolées pour exprimer des niveaux élevés de protéine de type facteur VIII ou des niveaux élevés à la fois de protéine de type facteur VIII et de protéine de type facteur von Willebrand (vWF), et dans lesquelles les CSM isolées sont isolées du tissu placentaire, et éventuellement dans lesquelles les CSM isolées sont isolées pendant la période prénatale à partir d'échantillons obtenus auprès de la mère du sujet ; et éventuellement
dans lesquelles les CSM modifiées sont générées en introduisant un vecteur viral dans les CSM isolées, le vecteur viral comprenant une séquence du gène du facteur VIII liée de manière opérationnelle à un promoteur constitutivement actif.

11. CSM destinées à être utilisées selon la revendication 10, dans lesquelles les CSM modifiées sont générées en modifiant les CSM isolées par édition génique d'une séquence endogène du gène du facteur VIII pour introduire une ou plusieurs modifications dans la séquence endogène du gène du facteur VIII qui augmentent l'expression de la protéine, la stabilité de la protéine, ou les deux, de la protéine de type facteur VIII.

12. CSM destinées à être utilisées selon l'une quelconque des revendications 10 à 11, dans lesquelles les CSM modifiées sont générées par introduction d'un vecteur viral dans les CSM isolées, le vecteur viral comprenant une séquence du gène vWF liée de manière opérationnelle à un promoteur constitutivement actif.

13. CSM destinées à être utilisées selon l'une quelconque des revendications 10 et 12, dans lesquelles les CSM modifiées sont générées par introduction d'un vecteur viral dans les CSM isolées, le vecteur viral comprenant une séquence du gène du facteur VIII liée de manière opérationnelle à un promoteur constitutivement actif et une séquence du gène vWF liée de manière opérationnelle à un promoteur constitutivement actif ; et éventuellement dans lesquelles la séquence du gène du facteur VIII et la séquence du gène vWF sont liées de manière opérationnelle au même promoteur constitutivement actif.

14. CSM destinées à être utilisées selon l'une quelconque des revendications 10 à 11, dans lesquelles les CSM modifiées sont générées par modification des CSM isolées par édition génique d'une séquence endogène du gène vWF pour introduire une ou plusieurs modifications dans la séquence endogène du gène vWF qui augmentent l'expression de la protéine, la stabilité de la protéine, ou les deux, de la protéine de type vWF.

15. CSM destinées à être utilisées selon l'une quelconque des revendications précédentes, dans lesquelles le procédé comprend l'injection des CSM modifiées dans le sujet *in utero* par injection intrapéritonéale ; et/ou dans lesquelles les CSM modifiées sont injectées au moins une fois, au moins deux fois ou au moins trois fois dans le sujet ; et/ou dans lesquelles les CSM modifiées sont injectées dans le sujet en une quantité allant d'environ 10⁷ à 10⁹ CSM par kilogramme de poids du sujet.
